# EUROPEAN PATENT SPECIFICATION

(11) **EP 2 347 744 B1**
(45) Date of publication and mention of the grant of the patent: **15.10.2014**
(21) Application number: 09826017.7
(22) Date of filing: 27.10.2009
(51) Int. Cl.: A61F 13/496, A61F 13/515

(54) **PULL-ON ABSORBENT ARTICLE**
ANZIEHBARER EINWEGARTIKEL
ARTICLE ABSORBENT DE TYPE A ENFILER

(30) Priority: 14.11.2008 JP 2008292324; 19.11.2008 JP 2008295573
(43) Date of publication of application: 27.07.2011
(73) Proprietor: Kao Corporation, Chuo-Ku Tokyo 103-8210 (JP)
(72) Inventor: SASAKI Jun, Haga-gun Tochigi 321-3497 (JP)
(74) Representative: Vossius & Partner
(86) International application number: PCT/JP2009/068370
(87) International publication number: WO 2010/055767

(56) References cited:
- EP-A1- 1 795 163
- EP-A1- 2 412 354
- JP-A- 2001 120 595
- JP-A- 2003 024 383
- JP-A- 2005 230 313
- JP-A- 2007 068 987
- US-A- 5 931 827
- US-A1- 2006 089 616

## Description

### Technical Field

The present invention relates to pull-on absorbent articles such as disposable diapers.

### Background Art

There have conventionally been proposed various pull-on absorbent articles of the type having a topsheet, a backsheet, and a liquid-retainable absorbent core, and including a pair of side-sealing sections being formed by fusion-bonding of respective overlapped sections where opposite side-edge sections of a stomach-side section and opposite side-edge sections of a back-side section overlap one another.

Further, Patent Document 1, for example, discloses a pull-on disposable diaper having three-dimensional waist gathers to prevent excrement from leaking from a waist opening of the diaper.

Furthermore, Patent Document 2, for example, discloses a pull-on disposable diaper having a waist ribbon that has elastic members enclosed inside and that is disposed along the entire perimeter of the waist opening to prevent excrement from leaking from a waist opening of the diaper.

Since both the disposable diapers disclosed in Patent Documents 1 and 2 employ either a waist ribbon or a constituent member that forms the three-dimensional waist gathers, the overlapped sections where the opposite side-edge sections of the stomach-side section and the opposite side-edge sections of the back-side section overlap one another are made up of a plurality of constituent members. Therefore, the basis weight in the overlapped section varies partially from region to region depending on the respective basis weights of the constituent materials and/or the positions where the constituent materials are disposed. Thus, when the side-sealing section is formed by fusion-bonding the overlapped section, the region in the side-sealing section having a large basis weight tends to become stiff. This may cause pain when the absorbent article is worn or may hurt the skin.

Further, since both the disposable diapers disclosed in Patent Documents 1 and 2 employ either a waist ribbon or a constituent member that forms the three-dimensional waist gathers, the number of constituent members that make up the overlapped sections--where the opposite side-edge sections of the stomach-side section and the opposite side-edge sections of the back-side section overlap one another--becomes large only in the region where the waist ribbon/constituent member is disposed. Therefore, when the side-sealing section is formed by fusion-bonding the overlapped section, the region of the side-sealing section in which the constituent member that forms the three-dimensional waist gathers or the waist ribbon is disposed tends to become stiff. This may cause pain when the absorbent article is worn or may hurt the skin.

Patent Document 1: Japanese Patent Laid Open JP-A-4-354948
Patent Document 2: Japanese Patent Laid Open JP-A-10-290818

EP 1 795 163 A1 discloses a pull-on absorbent article comprising a pair of side-sealing sections each being formed by fusion-bonding of respective overlapped sections. Opposite side-edge sections of a stomach-side section and opposite side-edge sections of a back-side section overlap one another. Each side-sealing section includes regions each having a different total basis weight of members that make up the overlapped section. Each of the overlapped sections is made up of a plurality of members having respective basis weights.

### Disclosure of the Invention

The present invention relates to a pull-on absorbent article in which its side-sealing sections are less prone to become stiff, even in cases where the basis weight of the overlapped sections where the opposite side-edge sections of the stomach-side section and the opposite side-edge sections of the back-side section overlap one another becomes partially large.

### Means for Solving the Problem

The present invention relates to a pull-on absorbent article including a pair of side-sealing sections each being formed by fusion-bonding of respective overlapped sections where opposite side-edge sections of a stomach-side section and opposite side-edge sections of a back-side section overlap one another. Each side-sealing section includes regions each having a different total basis weight of members that make up the overlapped section. In the pull-on absorbent article of the present invention, each of the overlapped sections is made up of a plurality of members having respective basis weights; and the larger a total basis weight of the respective basis weights of the plurality of the members is in a region of the side-sealing section, the smaller a fusion-bonded area is made in that region of the side-sealing section.

### Brief Description of the Drawings

[Fig. 1] Fig. 1 is a perspective of a pull-on disposable diaper according to a first embodiment of the present invention.
[Fig. 2] Fig. 2 is a developed plan view illustrating the pull-on disposable diaper of Fig. 1 in its developed, stretched-out state.
[Fig. 3] Fig. 3(a) is a cross-sectional view taken along line Y1-Y1 of Fig. 2, Fig. 3(b) is a cross-sectional view taken along line Y2-Y2 of Fig. 2, and Fig. 3(c) is an enlarged plan view of a side-sealing section.
[Fig. 4] Fig. 4 is a schematic diagram of an embossing roll for forming side-sealing sections of the pull-on disposable diaper illustrated in Fig. 1.
[Fig. 5] Fig. 5 is a developed plan view illustrating a pull-on disposable diaper according to a second embodiment of the present invention in its developed, stretched-out state.
[Fig. 6] Fig. 6(a) is a cross-sectional view taken along line Y1-Y1 of Fig. 5, Fig. 6(b) is a cross-sectional view taken along line Y2-Y2 of Fig. 5, and Fig. 6(c) is an enlarged plan view of a side-sealing section.
[Fig. 7] Fig. 7 is a developed plan view illustrating a pull-on disposable diaper according to another embodiment of the present invention in its developed, stretched-out state.
[Fig. 8] Fig. 8 is a developed plan view illustrating a pull-on disposable diaper according to another embodiment of the present invention in its developed, stretched-out state.
[Fig. 9] Fig. 9 is an enlarged plan view of a side-sealing section of a pull-on disposable diaper according to another embodiment of the present invention.
[Fig. 10] Fig. 10 is a perspective of a pull-on disposable diaper according to a third embodiment of the present invention.
[Fig. 11] Fig. 11 is a developed plan view illustrating the pull-on disposable diaper of Fig. 10 in its developed, stretched-out state.
[Fig. 12] Fig. 12(a) is a cross-sectional view taken along line Y1-Y1 of Fig. 11, Fig. 12(b) is a cross-sectional view taken along line Y2-Y2 of Fig. 11, and Fig. 12(c) is an enlarged plan view of a side-sealing section.
[Fig. 13] Fig. 13 is a schematic diagram of an embossing roll for forming side-sealing sections of the pull-on disposable diaper illustrated in Fig. 10.
[Fig. 14] Fig. 14 is a developed plan view illustrating a pull-on disposable diaper according to a fourth embodiment of the present invention in its developed, stretched-out state.
[Fig. 15] Fig. 15(a) is a cross-sectional view taken along line Y1-Y1 of Fig. 14, Fig. 15(b) is a cross-sectional view taken along line Y2-Y2 of Fig. 14, and Fig. 15(c) is an enlarged plan view of a side-sealing section.
[Fig. 16] Fig. 16 is a developed plan view illustrating a pull-on disposable diaper according to another embodiment of the present invention in its developed, stretched-out state.
[Fig. 17] Fig. 17 is a developed plan view illustrating a pull-on disposable diaper according to another embodiment of the present invention in its developed, stretched-out state.
[Fig. 18] Fig. 18 is an enlarged plan view of a side-sealing section of a pull-on disposable diaper according to another embodiment of the present invention.

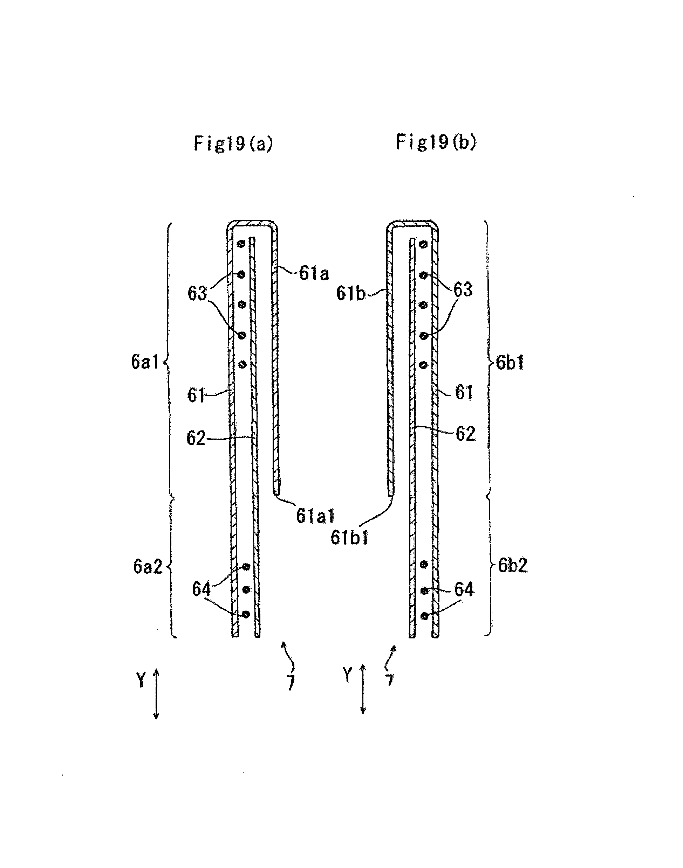

[Fig. 19] Fig. 19(a) is a cross-sectional view taken along line Y1-Y1 of a pull-on disposable diaper according to another embodiment of the present invention, and Fig. 19(b) is a cross-sectional view taken along line Y2-Y2 of the pull-on disposable diaper.

### Detailed Description of the Invention

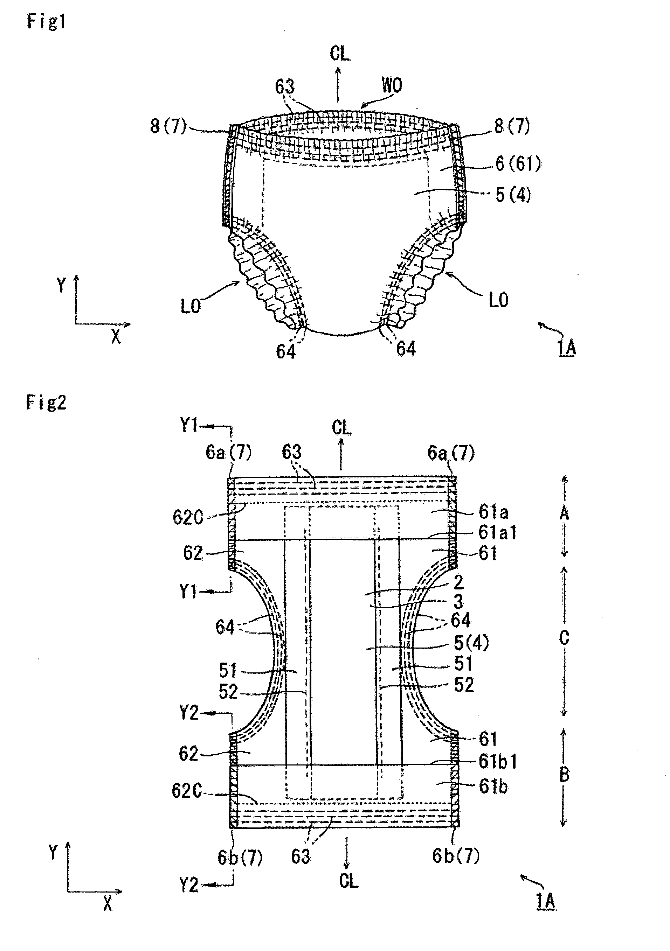

A preferred first embodiment of a pull-on disposable diaper, serving as a pull-on absorbent article of the present invention, is described below with reference to Figs. 1 to 4.

A pull-on disposable diaper 1A of the first embodiment includes a pair of side-sealing sections 8, 8 each being formed by fusion-bonding of respective overlapped sections 7, 7 where opposite side-edge sections 6a, 6a of a stomach-side section A and opposite side-edge sections 6b, 6b of a back-side section B overlap one another. Each overlapped section 7 is made up of a plurality of members having respective basis weights. Each of the side-sealing sections 8, 8 includes regions each having a different total basis weight of members that make up each overlapped section 7. In the pull-on disposable diaper 1A of the first embodiment, the larger the total basis weight of the respective basis weights of the plurality of the members is in a region of the side-sealing section 8, the smaller the fusion-bonded area is made in that region of the side-sealing section 8.

As illustrated in Figs. 1 and 2, the pull-on disposable diaper 1A of the first embodiment is formed to have line symmetry with respect to a center line CL. Accordingly, in the following description, the left-hand side will mainly be described for sections that have line symmetry.

The pull-on disposable diaper 1A of the first embodiment is described in detail. As illustrated in Fig. 2, the pull-on disposable diaper 1A (also referred to hereinafter as "diaper 1A") of the first embodiment includes: a substantially-elongated absorbent body 5 having a liquid-permeable topsheet 2, a liquid-impermeable (or water-repellent) backsheet 3, and an absorbent core 4 provided between the topsheet 2 and the backsheet 3; and an envelope 6 disposed on the backsheet side of the absorbent body 5 and attached and fixed thereto.

As illustrated in Fig. 2, the envelope 6 has a stomach-side section A, a crotch section C, and a back-side section B in its length direction (referred to hereinafter as "Y direction"; the Y direction is a direction parallel to the center line CL). A pair of side-sealing sections 8, 8 is formed by fusion-bonding respective overlapped sections 7, 7 where the opposite side-edge sections 6a, 6a of the stomach-side section A of the envelope 6 and the opposite side-edge sections 6b, 6b of the back-side section B of the envelope 6 overlap one another, thus forming a pull-on disposable diaper 1A having a waist opening WO and a pair of leg openings LO, LO, as illustrated in Fig. 1. The stomach-side section A of the envelope 6 is a section to be located on the wearer's stomach side when the diaper 1A is worn, the back-side section B is a section to be located on the wearer's back side, and the crotch section C is a section to be located in the wearer's crotch.

The envelope 6 extends over the entire outer region of the absorbent body 5, and includes an outer-layer sheet 61 forming an outer surface of the diaper, an inner-layer sheet 62 disposed on the inner side of the outer-layer sheet 61, and folded sections 61a, 61b or 62a, 62b formed by folding, toward the inner-layer sheet 62, outward-extending portions of the outer-layer sheet 61 or the inner-layer sheet 62 that extend outward lengthwise (Y-direction-wise) from the absorbent body 5. As illustrated in Fig. 2, the opposite side edges of the envelope 6 of the present diaper 1A are constricted inward at the lengthwise (Y-direction-wise) central section. Further, as illustrated in Figs. 1 to 3, the envelope 6 is made up of the outer-layer sheet 61 forming an outer surface of the diaper, the inner-layer sheet 62 disposed on the inner side of the outer-layer sheet 61, a plurality of waist elastic members 63 disposed and fixed between the outer-layer sheet 61 and the folded section 61a formed by folding the outer-layer sheet 61, and a plurality of leg elastic members 64 disposed and fixed between the outer-layer sheet 61 and the inner-layer sheet. The plurality of waist elastic members 63 are provided along the circumferential edge section of the stomach-side section A and the back-side section B which becomes the waist opening WO, and are disposed and fixed along the width direction (referred to hereinafter as "X direction"; the X direction is a direction perpendicular to the center line CL extending in the length direction of the disposable diaper 1A) in their stretched state. The plurality of leg elastic members 64 are disposed along the circumferential edge sections that become the leg openings LO, as illustrated in Fig. 2, and are disposed and fixed in their stretched state.

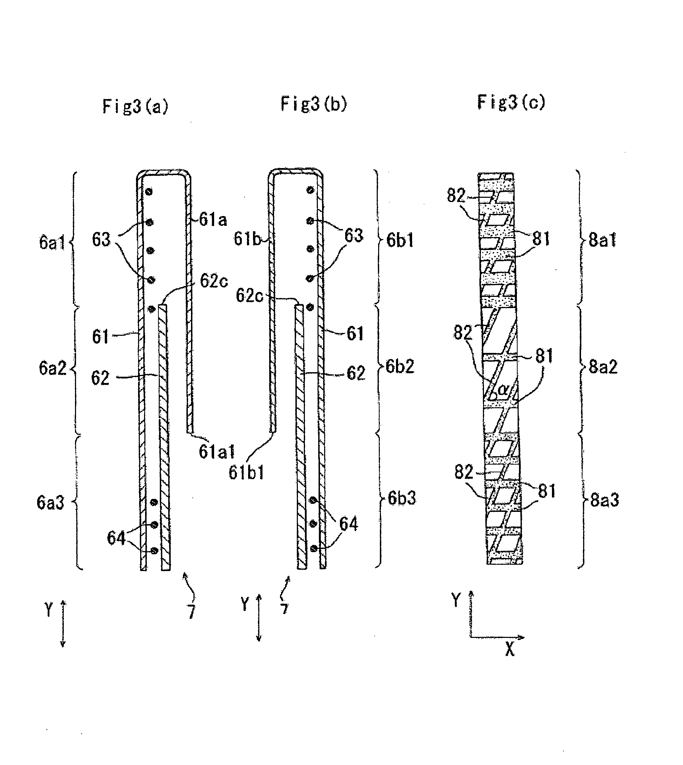

The outer-layer sheet 61 of the envelope 6 of the diaper 1A has, in both the stomach-side section A and the back-side section B, a length that extends further outward lengthwise (Y-direction-wise) from the opposite lengthwise (Y-direction-wise) ends 62c, 62c of the inner-layer sheet 62 as illustrated in Fig. 2, Fig. 3(a), and Fig. 3(b). Folding back the extending portions toward the inner-layer sheet 62 will form the folded sections 61a, 61b. In the stomach-side section A, the outer-layer sheet 61 and the folded section 61 a sandwich the plurality of waist elastic members 63 to fix these therebetween, as illustrated in Fig. 2 and Fig. 3(a). Likewise, in the back-side section B, the outer-layer sheet 61 and the folded section 61b sandwich the plurality of waist elastic members 63 to fix these therebetween, as illustrated in Fig. 2 and Fig. 3(b). As illustrated in Fig. 2 and Fig. 3(a), the lengthwise (Y-direction-wise) end 61 a1 of the folded section 61a extends beyond the lengthwise (Y-direction-wise) end of the absorbent body 5 on the side of the stomach-side section A and more toward the crotch section C. Likewise, as illustrated in Fig. 2 and Fig. 3(b), the lengthwise (Y-direction-wise) end 61b1 of the folded section 61b extends beyond the lengthwise (Y-direction-wise) end of the absorbent body 5 on the side of the back-side section B and more toward the crotch section C. In this way, the opposite lengthwise (Y-direction-wise) ends of the absorbent body 5 are respectively sandwiched between the envelope 6 and the respective folded sections 61a, 61b.

The inner-layer sheet 62 of the envelope 6 of the diaper 1A is disposed so that, in both the stomach-side section A and the back-side section B, the opposite lengthwise (Y-direction-wise) ends 62c, 62c thereof are located near the opposite lengthwise (Y-direction-wise) ends of the absorbent body 5, as illustrated in Fig. 2, Fig. 3(a), and Fig. 3(b). That is, different from the outer-layer sheet 61, the inner-layer sheet 62 does not have folded sections as illustrated in Fig. 3(a) and Fig. 3(b), and the length of the inner-layer sheet 62 in the length direction (Y direction) is made shorter than the length of the outer-layer sheet 61 in the length direction (Y direction).

In the present diaper 1A, the inner-layer sheet 62 used in the envelope 6 has a larger basis weight than that of the outer-layer sheet 61. From the standpoint of texture and cost, it is preferable that in the present diaper 1A, the basis weight of the outer-layer sheet 61 is 8 g/m² to 30 g/m², and the basis weight of the inner-layer sheet 62 is 8 g/m² to 30 g/m². The basis weight is measured as follows.

### Method for Measuring Basis Weight

A measurement piece that is at least 30 mm wide and 30 mm long is cut out from each of the inner-layer sheet 62 and the outer-layer sheet 61, to obtain measurement pieces each having a total area of 900 mm² from a single diaper. The measurement piece is weighed using an electronic balance having a minimum display of 1 mg, and the measured value is converted into basis weight. An average value of measurement pieces sampled from five (n = 5) diapers is regarded as the basis weight value. Note that the measurement pieces of the inner-layer sheet 62 and the outer-layer sheet 61 are to be sampled from sections except for the side-sealing sections 8. In cases where it is not possible to sample a measurement piece that is 30 mm wide and 30 mm long, a piece having the largest possible area should be sampled, and the basis weight can be obtained through conversion based on the area thereof. In cases where the inner-layer sheet and the outer-layer sheet are joined using an adhesive such as a hot-melt adhesive, the weight should be measured after washing off the adhesive with an organic solvent, such as chloroform, and then thoroughly drying the piece. The method above is to be used for measuring the basis weight of members other than the members that form the envelope 6.

As illustrated in Figs. 1 and 2, the overlapped section 7 of the diaper 1A is formed of the envelope 6 which is made up of a plurality of members. More specifically, as illustrated in Fig. 3(a), each side-edge section 6a of the stomach-side section A includes the outer-layer sheet 61, the inner-layer sheet 62, and the folded section 61a, and as illustrated in Fig. 3(b), each side-edge section 6b of the back-side section B includes the outer-layer sheet 61, the inner-layer sheet 62, and the folded section 61b. The section where the side-edge section 6a of the stomach-side section A and the side-edge section 6b of the back-side section B overlap one another becomes the overlapped section 7, as illustrated in Fig. 2.

As illustrated in Fig. 3(a), in the present diaper 1A, the side-edge section 6a of the stomach-side section A has, in the length direction (Y direction): a region 6a1 made up of two members, i.e., the outer-layer sheet 61 and the folded section 61 a; a region 6a2 made up of three members, i.e., the outer-layer sheet 61, the inner-layer sheet 62, and the folded section 61a; and a region 6a3 made up of two members, i.e., the outer-layer sheet 61 and the inner-layer sheet 62. Note that the basis weight of the inner-layer sheet 62 is larger than that of the outer-layer sheet 61. Therefore, as regards the total basis weight of the respective basis weights of the plurality of the members in each region, the total basis weight of the region 6a2 made up of three members is larger than both the total basis weight of the region 6a1 made up of two members and the total basis weight of the region 6a3 made up of two members, and further, the total basis weight of the region 6a3 made up of two members including the inner-layer sheet 62 is larger than the total basis weight of the region 6a1 made up of two members but not including the inner-layer sheet 62.

As illustrated in Fig. 3(b), in the present diaper 1A, the side-edge section 6b of the back-side section B similarly has, in the length direction (Y direction): a region 6b1 made up of two members, i.e., the outer-layer sheet 61 and the folded section 61b; a region 6b2 made up of three members, i.e., the outer-layer sheet 61, the inner-layer sheet 62, and the folded section 61b; and a region 6b3 made up of two members, i.e., the outer-layer sheet 61 and the inner-layer sheet 62. Note that the basis weight of the inner-layer sheet 62 is larger than that of the outer-layer sheet 61. Therefore, as regards the total basis weight of the respective basis weights of the plurality of the members in each region, the total basis weight of the region 6b2 made up of three members is larger than both the total basis weight of the region 6b1 made up of two members and the total basis weight of the region 6b3 made up of two members, and further, the total basis weight of the region 6b3 made up of two members including the inner-layer sheet 62 is larger than the total basis weight of the region 6b1 made up of two members but not including the inner-layer sheet 62. Each overlapped section 7 where the side-edge section 6a and the side-edge section 6b overlap one another includes a region having the largest total basis weight where the region 6a2 and the region 6b2 overlap one another, a region having the next largest total basis weight where the region 6a3 and the region 6b3 overlap one another, and a region having the smallest total basis weight where the region 6a1 and the region 6b1 overlap one another. Fusion-bonding this overlapped section 7 will form the side-sealing section 8.

The side-sealing section 8 is formed such that, the larger the total basis weight of the respective basis weights of the plurality of the members making up the overlapped section 7 is in a region, the smaller the fusion-bonded area is in that region. As illustrated in Fig. 3(c), in the side-sealing section 8 of the diaper 1A, the fusion-bonded area in a sealing region 8a2 formed by fusion-bonding the region where the region 6a2 and the region 6b2 overlap one another is made smaller than both the fusion-bonded area in a sealing region 8a3 formed by fusion-bonding the region where the region 6a3 and the region 6b3 overlap one another and the fusion-bonded area in a sealing region 8a1 formed by fusion-bonding the region where the region 6a1 and the region 6b1 overlap one another, and further, the fusion-bonded area in the sealing region 8a3 formed by fusion-bonding the region where the region 6a3 and the region 6b3 overlap one another is made smaller than the fusion-bonded area in the sealing region 8a1 formed by fusion-bonding the region where the region 6a1 and the region 6b1 overlap one another. More specifically, as illustrated in Fig. 3(c), each of the sealing region 8a1, the sealing region 8a2, and the sealing region 8a3 includes fusion-bonded sections 81 extending along the width direction (X direction) and fusion-bonded sections 82 extending obliquely toward the center of the diaper 1A's lengthwise (Y-direction-wise) end on the side of the stomach-side section A. The width of each fusion-bonded section 81 in the sealing region 8a2 is made narrower than the width of each fusion-bonded section 81 in both the sealing region 8a1 and the sealing region 8a3, and further, the width of each fusion-bonded section 81 in the sealing region 8a3 is made narrower than the width of each fusion-bonded section 81 in the sealing region 8a1. The width of the fusion-bonded section 82 is substantially the same in all of the sealing region 8a1, the sealing region 8a2, and the sealing region 8a3, and the distance between adjacent fusion-bonded sections 82 is also substantially the same.

The width of each fusion-bonded section 81 in the sealing region 8a2 is preferably 0.2 mm to 2 mm, the width of each fusion-bonded section 81 in the sealing region 8a3 is preferably 0.4 mm to 2.5 mm, and the width of each fusion-bonded section 81 in the sealing region 8a1 is preferably 0.6 mm to 3 mm. The distance between adjacent fusion-bonded sections 81 in the sealing region 8a2 is preferably 3 mm to 8 mm, the distance between adjacent fusion-bonded sections 81 in the sealing region 8a3 is preferably 2 mm to 7 mm, and the distance between adjacent fusion-bonded sections 81 in the sealing region 8a1 is preferably 1 mm to 6 mm. The angle α illustrated in Fig. 3(c) at which the fusion-bonded section 81 intersects with the fusion-bonded section 82 is preferably 30° to 70°. The width of each fusion-bonded section 82 in the sealing region 8a1, the sealing region 8a2, and the sealing region 8a3 is preferably 0.5 mm to 3 mm, and the distance between adjacent fusion-bonded sections 82 is preferably 1 mm to 5 mm.

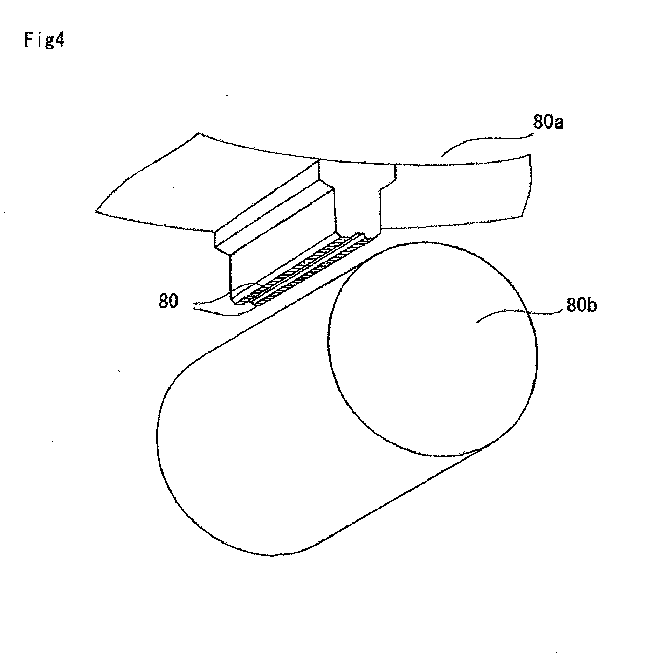

Fusion-bonding of the side-sealing section 8 is achieved by employing, for example, heat embossing, ultrasonic embossing, or high-frequency embossing. In the present diaper 1A, a pair of ultrasonic embossing rolls 80a, 80b is employed, as illustrated in Fig. 4. More specifically, a continuous strip of diapers 1A is passed between an embossing roll 80a and a flat roll 80b, wherein the embossing roll 80a has projections 80 shaped so as to correspond to the shape of the side-sealing sections 8 illustrated in Fig. 3(c), and the flat roll 80b has a flat surface. The projections 80 and the flat roll 80b perform embossing at the position of the overlapped section 7 where the side-edge section 6a and the side-edge section 6b overlap one another, to thus form a side-sealing section 8. Each projection 80 includes projecting pieces that correspond to the respective shapes of the sealing region 8a1, the sealing region 8a2, and the sealing region 8a3, wherein the projecting pieces are arranged in a direction orthogonal to the flow direction of the continuous strip of diapers 1A such that the direction orthogonal to the flow direction matches the length direction (Y direction) of the side-sealing section 8 illustrated in Fig. 3(c). Accordingly, the side-sealing section 8 is formed by a single embossing process.

Herein, the "fusion-bonded area of the sealing region 8a1", the "fusion-bonded area of the sealing region 8a2", and the "fusion-bonded area of the sealing region 8a3" each refer to the area of portions embossed by the projection 80, and more specifically, refer to the bottom-surface area of embossed portions that have been turned into a film by the embossing process when viewing the side-sealing section 8 from above. Note that in cases where the side-sealing section 8 has shrunk in the width direction (X direction) etc. by means of the waist elastic members 63 etc., the portion's bottom-surface area is to be measured in its stretched-out state.

It is preferable that the fusion-bond strength of the side-sealing section 8 is 5 N/30 mm to 40 N/30 mm, and more preferably 5 N/30 mm to 30 N/30 mm. The "fusion-bond strength of the side-sealing section 8" means that the fusion-bond strength in each of the sealing region 8a1, the sealing region 8a2, and the sealing region 8a3 falls within the above numerical range. Setting the fusion-bond strength of the side-sealing section 8 within the numerical range of 5 N/30 mm to 40 N/30 mm will prevent the side-sealing sections 8 from peeling apart during use, but will allow the side-sealing sections 8 to be torn easily after use to thus facilitate diaper change.

It is preferable that a ratio (Fmin/Fmax) of the minimum fusion-bond strength of the side-sealing section 8 to the maximum fusion-bond strength of the side-sealing section is 2/3 < Fmin/Fmax ≤ 1, and more preferably 5/6 < Fmin/Fmax ≤ 1. Setting the ratio (Fmin/Fmax) of the minimum fusion-bond strength of the side-sealing section 8 to the maximum fusion-bond strength of the side-sealing section within the numerical range of 2/3 < Fmin/Fmax ≤ 1 will allow the side-sealing sections 8 to be torn easily after use with uniform force because of the small variation in fusion-bond strength, thus further facilitating diaper change. The fusion-bond strength is measured as follows.

### Method for Measuring Fusion-bond Strength

The side-sealing section 8 is cut out from a diaper 1A along the length direction (Y direction) thereof. Next, the cut-out side-sealing section 8 is cut widthwise (X-direction-wise) in the sealing region 8a1, the sealing region 8a2, and the sealing region 8a3 to obtain a sample of the sealing region 8a1, a sample of the sealing region 8a2, and a sample of the sealing region 8a3, the lengthwise (Y-direction-wise) length of the sealing section of each sample being 30 mm.

The sealing strength in each region is measured using a tensile tester (Autograph AGS50A (trade name) manufactured by Shimadzu Corporation). Measurement is to be performed at a tensile speed of 300 mm/min to determine the maximum strength. Measurement is to be performed five (n = 5) times, and an average value thereof is regarded as the fusion-bond strength for each region. In cases where it is not possible to cut out a sample with a sealing-section length of 30 mm from each region, the fusion-bond strength can be obtained by performing measurement with the largest-possible length closest to 30 mm and converting the measurement value to a value for the 30-mm length.

As illustrated in Fig. 2, the absorbent body 5 is substantially elongated and has a liquid-permeable topsheet 2, a liquid-impermeable (or water-repellent) backsheet 3, and a liquid-retainable absorbent core 4 provided between the topsheet 2 and the backsheet 3. As illustrated in Fig. 2, the absorbent body 5 is disposed from the back-side section B of the envelope 6 to the stomach-side section A thereof, and the opposite lengthwise (Y-direction-wise) ends of the absorbent body 5 are located at positions setting back inward in the length direction (Y direction) from the respective opposite lengthwise (Y-direction-wise) ends of the envelope 6. The absorbent body 5 is joined to the inner-layer sheet 62 of the envelope 6 through joining such as by heat sealing or ultrasonic sealing, with an adhesive, and the like.

As illustrated in Fig. 2, the opposite lengthwise (Y-direction-wise) side sections of the absorbent body 5 have side cuffs 51, 51 made of a liquid-resistible/water-repellent and breathable material. An elastic member 52 for forming the side cuff is disposed in its stretched state along the length direction (Y direction) in the vicinity of the free end of each side cuff 51 and fixed thereto. When the diaper is worn, the respective free-end sides of the side cuffs 51 stand up, thus preventing excrement from flowing out from the absorbent body 5 in the width direction (X direction). Each sheet for forming the side cuff 51 is fixed such that a portion thereof located on the outer side, in the width direction (X direction) of the absorbent body 5, and having a predetermined width is tucked in toward the skin-non-contacting side.

Materials for forming the pull-on disposable diaper 1A of the first embodiment are described below.

It is preferable that the fusion-bonding component of the members that make up the overlapped section 7 is made of a general-purpose resin, such as polypropylene, polyethylene, or polyester, in terms of cost, and it is preferable that the respective fusion-bonding components of the members that make up the overlapped section 7 are components of the same series. In the present diaper 1A, the members that make up the overlapped section 7 are the outer-layer sheet 61 (including the folded section 61 a) and the inner-layer sheet 62 (including the folded section 62a). Accordingly, it is preferable that the outer-layer sheet 61 and the inner-layer sheet 62 are of components of the same series. "Components of the same series as polypropylene" serving as respective fusion-bonding components of the outer-layer sheet 61 and the inner-layer sheet 62 include, for example: nonwoven sheets made only of homo-PP fibers containing polypropylene monomers; nonwoven sheets made only of random-PP fibers in which a small amount of ethylene etc. is introduced randomly into the propylene chain; nonwoven sheets made of sheath/core conjugate fibers or side-by-side conjugate fibers that contain 50% or more of homo-PP resin or random-PP resin and also contain thermoplastic resin other than homo-PP resin or random-PP resin (such as polyethylene resin, polyester resin, etc.); and nonwoven sheets made of fibers that contain 50% or more of homo-PP fibers or random-PP fibers and also contain fibers other than homo-PP fibers or random-PP fibers (such as rayon fiber, cotton fiber, polyethylene fiber, polyester fiber, etc.).

The phrase "respective fusion-bonding components of the outer-layer sheet 61 and the inner-layer sheet 62 are components of the same series as polyethylene or polyester" can be construed similarly as the "components of the same series as polypropylene". Particularly, "components of the same series as polyethylene" preferably include, for example, nonwoven sheets made of fibers admixed with sheath/core conjugate fibers or side-by-side conjugate fibers containing polyester resin as the core and polyethylene resin as the sheath and/or admixed with sheath/core conjugate fibers or side-by-side conjugate fibers containing polypropylene resin as the core and polyethylene resin as the sheath. Further, particularly, "components of the same series as polyester" preferably include, for example, nonwoven sheets made of fibers admixed with sheath/core conjugate fibers or side-by-side conjugate fibers containing polyester resin as the core and low-melting-point polyester resin as the sheath. Composing the respective fusion-bonding components of the members that make up the overlapped section 7 out of components of the same series as polypropylene, polyethylene, or polyester provides good compatibility among the fusion-bonding components as well as favorable fusion-bond strength, thereby allowing fusion-bonding to be performed at lower temperatures. Because embossing does not have to be performed at high temperatures, portions other than the embossed portions will not stiffen, thus making the side-sealing sections soft as a whole and improving the feel against the skin.

Any kind of material generally used for absorbent articles, such as disposable diapers, can be used without particular limitation for the topsheet 2, the backsheet 3, and the sheets for forming the side cuffs 51. For the topsheet 2, it is possible to use, for example, a liquid-permeable nonwoven fabric, a porous film, or a laminate thereof. For the backsheet 3, it is possible to use, for example, a resin film or a laminate of a resin film and a nonwoven fabric. For the sheets for forming the side cuffs 51, it is possible to use, for example, stretchable films, nonwoven fabrics, woven fabrics, or laminated sheets made of the above.

Any kind of material generally used for absorbent articles, such as disposable diapers, can be used without particular limitation for the absorbent core 4. For example, for the absorbent core 4, it is possible to use either a fiber aggregate made of fibrous material such as pulp and wrapped with a covering such as tissue paper or a permeable nonwoven fabric, or the above fiber aggregate further containing high-absorbent polymer particles and wrapped with a like covering.

Any kind of material generally used for absorbent articles, such as disposable diapers, can be used without particular limitation for the elastic members 52 for forming the side cuffs, the waist elastic members 63, and the leg elastic members 64. For example, it is possible to use stretchable materials made of natural rubber, polyurethane, polystyrene-polyisoprene copolymer, polystyrene-polybutadiene copolymer, or polyethylene-α-olefin copolymers such as ethyl acrylate-ethylene.

The following describes effects achieved by using the pull-on disposable diaper 1A of the foregoing first embodiment of the present invention.

As illustrated in Figs. 1 to 3, in the diaper 1A of the first embodiment, the larger the total basis weight of the respective basis weights of the plurality of the members that make up each of the overlapped sections 7, 7 is in a region of the side-sealing section 8, the smaller the fusion-bonded area is made in that region of the side-sealing section 8. In general, as the total basis weight of the members forming the overlapped section increases, the amount of fusion-bonded resin also increases along therewith. Therefore, the portion with the increased resin amount becomes stiffer when that portion is thermally resinified. Such stiff portions may hurt the skin or cause other problems when the article is worn. In contrast, in the present invention, the fusion-bonded area is varied in accordance with the total basis weight and the total fusion-bonded area of the entire side-sealing section 8 can thus be kept within certain bounds, even in cases where the total basis weight of the plurality of members that make up the overlapped sections 7, 7--where the opposite side-edge sections 6a, 6a of the stomach-side section A and the opposite side-edge sections 6b, 6b of the back-side section B overlap one another--becomes partially large and the amount of resin that constitutes the fusion-bonding components also becomes large, as described above. Thus, the side-sealing sections 8 are less prone to become stiff and thus less prone to cause pain when the absorbent article is worn or to hurt the skin. Furthermore, the fusion-bond strength in each region becomes substantially the same. Thus, when the side sections are to be peeled apart after use, the peeling can be done with a uniform force, thereby improving usability.

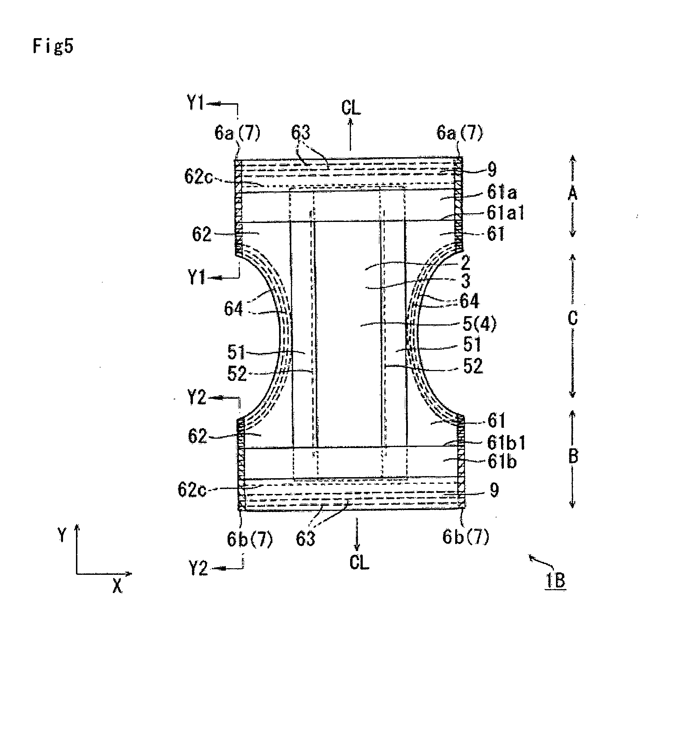

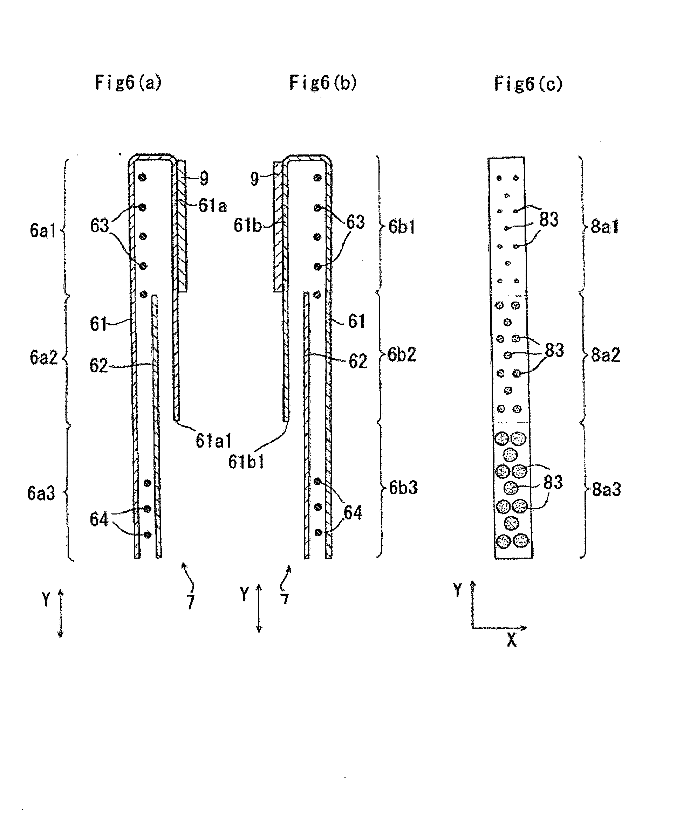

Next, a pull-on disposable diaper according to a second embodiment of the pull-on absorbent article of the present invention is described below with reference to Figs. 5 and 6.

The following mainly describes features of the pull-on disposable diaper 1B of the second embodiment that are different from the pull-on disposable diaper 1A of the foregoing first embodiment, and features in common are accompanied with the same symbols and are omitted from explanation. The explanation on the pull-on disposable diaper 1A of the first embodiment applies as appropriate to features that are not particularly explained below.

In the pull-on disposable diaper 1B (also referred to hereinafter as "diaper 1B") of the second embodiment, the overlapped section 7 is configured to include a member, such as a sweat-absorbent sheet, besides the members forming the envelope 6. More specifically, as illustrated in Figs. 5 and 6, the diaper 1B differs greatly from the diaper 1A in that it has a sweat-absorbent sheet 9 extending in the width direction (X direction) on the stomach-side section A and back-side section B. The diaper 1B is described in detail below.

As illustrated in Fig. 5, the envelope 6 of the diaper 1B extends over the entire outer region of the absorbent body 5, and includes an outer-layer sheet 61 forming an outer surface of the diaper, an inner-layer sheet 62 disposed on the inner side of the outer-layer sheet 61, and folded sections 61a, 61b formed by folding, toward the inner-layer sheet 62, outward-extending portions of the outer-layer sheet 61 that extend outward lengthwise (Y-direction-wise) from the absorbent body 5. The outer-layer sheet 61 of the envelope 6 of the diaper 1B has, in both the stomach-side section A and the back-side section B, a length that extends further outward lengthwise (Y-direction-wise) from sections where the waist elastic members 63 are sandwiched and fixed between the outer-layer sheet 61 and the inner-layer sheet 62 as illustrated in Figs. 5 and 6. Folding back the extending portions toward the inner-layer sheet 62 will form the folded sections 61a, 61b. As illustrated in Fig. 5 and Fig. 6(a), the lengthwise (Y-direction-wise) end 61a1 of the folded section 61a extends beyond the lengthwise (Y-direction-wise) end of the absorbent body 5 on the side of the stomach-side section A and more toward the crotch section C. Likewise, as illustrated in Fig. 5 and Fig. 6(b), the lengthwise (Y-direction-wise) end 61b1 of the folded section 61b extends beyond the lengthwise (Y-direction-wise) end of the absorbent body 5 on the side of the back-side section B and more toward the crotch section C. In this way, the opposite lengthwise (Y-direction-wise) ends of the absorbent body 5 are respectively sandwiched between the envelope 6 and the respective folded sections 61a, 61b.

The inner-layer sheet 62 of the envelope 6 of the diaper 1B is disposed so that, in both the stomach-side section A and the back-side section B, the opposite lengthwise (Y-direction-wise) ends 62c, 62c thereof are located near the opposite lengthwise (Y-direction-wise) ends of the absorbent body 5, as illustrated in Fig. 5, Fig. 6(a), and Fig. 6(b). That is, different from the outer-layer sheet 61, the inner-layer sheet 62 does not have folded sections as illustrated in Fig. 6(a) and Fig. 6(b), and the length of the inner-layer sheet 62 in the length direction (Y direction) is made shorter than the length of the outer-layer sheet 61 in the length direction (Y direction).

As illustrated in Fig. 5, Fig. 6(a), and Fig. 6(b), the sweat-absorbent sheets 9 of the diaper 1B are disposed extending in the width direction (X direction) respectively on the stomach-side section A and the back-side section B. The sweat-absorbent sheet 9 is disposed on the folded section 61a, 61b of the outer-layer sheet 61 and spans the opposite side-edge sections 6a, 6a of the stomach-side section A and the opposite side-edge sections 6b, 6b of the back-side section B. The lengthwise (Y-direction-wise) length of the sweat-absorbent sheet 9 on the stomach-side section A extends from the vicinity of the diaper 1B's lengthwise (Y-direction-wise) end in the stomach-side section A to the vicinity of the inner-layer sheet 62's lengthwise (Y-direction-wise) end in the stomach-side section A. Likewise, the lengthwise (Y-direction-wise) length of the sweat-absorbent sheet 9 on the back-side section B extends from the vicinity of the diaper 1B's lengthwise (Y-direction-wise) end in the back-side section B to the vicinity of the inner-layer sheet 62's lengthwise (Y-direction-wise) end in the back-side section B.

In the present diaper 1B, the basis weight of the inner-layer sheet 62 of the envelope 6 and the basis weight of the outer-layer sheet 61 are substantially the same, and the sweat-absorbent sheet 9 used herein has a larger basis weight than those of the inner-layer sheet 62 and the outer-layer sheet 61. From the standpoint of sweat absorbency, it is preferable that the basis weight of the sweat-absorbent sheet 9 is 10 g/m² to 40 g/m². From the standpoint of texture and cost, it is preferable that in the present diaper 1B, the basis weight of the outer-layer sheet 61 and the basis weight of the inner-layer sheet 62 are 8 g/m² to 30 g/m². The basis weight of the sweat-absorbent sheet 9 is measured according to the Method for Measuring Basis Weight described further above for measurement of the basis weight of the outer-layer sheet 61 and the inner-layer sheet 62.

As illustrated in Fig. 6(a), each side-edge section 6a of the stomach-side section A of the diaper 1B includes the outer-layer sheet 61, the inner-layer sheet 62, the folded section 61 a, and the sweat-absorbent sheet 9, and as illustrated in Fig. 6(b), each side-edge section 6b of the back-side section B includes the outer-layer sheet 61, the inner-layer sheet 62, the folded section 61b, and the sweat-absorbent sheet 9.

As illustrated in Fig. 6(a), in the present diaper 1B, the side-edge section 6a of the stomach-side section A has, in the length direction (Y direction): a region 6a1 made up of three members, i.e., the outer-layer sheet 61, the folded section 61a, and the sweat-absorbent sheet 9; a region 6a2 made up of three members, i.e., the outer-layer sheet 61, the inner-layer sheet 62, and the folded section 61a; and a region 6a3 made up of two members, i.e., the outer-layer sheet 61 and the inner-layer sheet 62. Note that the basis weight of the sweat-absorbent sheet 9 is larger than those of the outer-layer sheet 61 and the inner-layer sheet 62, and the basis weight of the outer-layer sheet 61 and the basis weight of the inner-layer sheet 62 are substantially the same. Therefore, as regards the total basis weight of the respective basis weights of the plurality of the members in each region, the total basis weight of the region 6a1 is larger than both the total basis weight of the region 6a2 and the total basis weight of the region 6a3, and further, the total basis weight of the region 6a2 is larger than the total basis weight of the region 6a3.

As illustrated in Fig. 6(b), in the present diaper 1B, the side-edge section 6b of the back-side section B similarly has, in the length direction (Y direction): a region 6b1 made up of three members, i.e., the outer-layer sheet 61, the folded section 61b, and the sweat-absorbent sheet 9; a region 6b2 made up of three members, i.e., the outer-layer sheet 61, the inner-layer sheet 62, and the folded section 61b; and a region 6b3 made up of two members, i.e., the outer-layer sheet 61 and the inner-layer sheet 62. Note that the basis weight of the sweat-absorbent sheet 9 is larger than those of the outer-layer sheet 61 and the inner-layer sheet 62, and the basis weight of the outer-layer sheet 61 and the basis weight of the inner-layer sheet 62 are substantially the same. Therefore, as regards the total basis weight of the respective basis weights of the plurality of the members in each region, the total basis weight of the region 6b1 is larger than both the total basis weight of the region 6b2 and the total basis weight of the region 6b3, and further, the total basis weight of the region 6b2 is larger than the total basis weight of the region 6b3. Each overlapped section 7 where the side-edge section 6a and the side-edge section 6b overlap one another includes a region having the largest total basis weight where the region 6a1 and the region 6b1 overlap one another, a region having the next largest total basis weight where the region 6a2 and the region 6b2 overlap one another, and a region having the smallest total basis weight where the region 6a3 and the region 6b3 overlap one another. Fusion-bonding this overlapped section 7 will form the side-sealing section 8.

The side-sealing section 8 is formed such that, the larger the total basis weight of the respective basis weights of the plurality of the members making up the overlapped section 7 is in a region, the smaller the fusion-bonded area is made in that region. As illustrated in Fig. 6(c), in the side-sealing section 8 of the diaper 1B, the fusion-bonded area in a sealing region 8a1 formed by fusion-bonding the region where the region 6a1 and the region 6b1 overlap one another is made smaller than both the fusion-bonded area in a sealing region 8a2 formed by fusion-bonding the region where the region 6a2 and the region 6b2 overlap one another and the fusion-bonded area in a sealing region 8a3 formed by fusion-bonding the region where the region 6a3 and the region 6b3 overlap one another, and further, the fusion-bonded area in the sealing region 8a2 formed by fusion-bonding the region where the region 6a2 and the region 6b2 overlap one another is made smaller than the fusion-bonded area in the sealing region 8a3 formed by fusion-bonding the region where the region 6a3 and the region 6b3 overlap one another. More specifically, as illustrated in Fig. 6(c), each of the sealing region 8a1, the sealing region 8a2, and the sealing region 8a3 includes a plurality of circular fusion-bonded sections 83. The diameter of each fusion-bonded section 83 in the sealing region 8a1 is made shorter than the diameter of each fusion-bonded section 83 in the sealing region 8a2 and the sealing region 8a3, and the diameter of each fusion-bonded section 83 in the sealing region 8a2 is made shorter than the diameter of each fusion-bonded section 83 in the sealing region 8a3.

The diameter of each fusion-bonded section 83 in the sealing region 8a1 is preferably 1 mm to 3 mm. The diameter of each fusion-bonded section 83 in the sealing region 8a2 is preferably 2 mm to 4 mm. The diameter of each fusion-bonded section 83 in the sealing region 8a3 is preferably 3 mm to 5 mm.

Materials for forming the pull-on disposable diaper 1B of the second embodiment are described below.

Only features of the pull-on disposable diaper 1B of the second embodiment that are different from those of the pull-on disposable diaper 1A of the first embodiment are described below. Features that are not specifically described are the same as those in the pull-on disposable diaper 1A of the first embodiment.

Any kind of material generally used for absorbent articles, such as disposable diapers, can be used without particular limitation for the sweat-absorbent sheet 9. For example, for the sweat-absorbent sheet 9, it is possible to use a spunlace nonwoven fabric, an air-through nonwoven fabric, paper, or the like that contains at least 50% by weight, and more preferably at least 60% by weight, of hydrophilic fibers such as rayon, cotton, or pulp.

The following describes effects achieved by using the pull-on disposable diaper 1B of the foregoing second embodiment of the present invention.

The pull-on disposable diaper 1B of the second embodiment achieves similar effects as those of the pull-on disposable diaper 1A of the first embodiment. The following describes effects thereof that differ from those of the pull-on disposable diaper 1 A of the first embodiment.

As illustrated in Figs. 5 and 6, the disposable diaper 1B of the second embodiment has, in particular, sweat-absorbent sheets 9 provided respectively on the stomach-side section A and the back-side section B. Therefore, the total basis weight of the respective basis weights of the plurality of the members that make up each of the overlapped sections 7, 7--where the opposite side-edge sections 6a, 6a of the stomach-side section A and the opposite side-edge sections 6b, 6b of the back-side section B overlap one another--becomes partially large. However, since the fusion-bonded area is varied in accordance with the total basis weight, the total fusion-bonded area of the entire side-sealing section 8 can be kept within certain bounds. Thus, the side-sealing sections 8 are less prone to become stiff and thus less prone to cause pain when the absorbent article is worn or to hurt the skin.

The pull-on absorbent article of the present invention is not limited whatsoever to the pull-on disposable diapers 1A, 1B of the foregoing first and second embodiments, but can be modified as appropriate. Further, the constituent features of the pull-on disposable diapers 1A, 1B of the foregoing first and second embodiments can be worked in combination as appropriate, as long as such combined use does not contradict the gist of the invention.

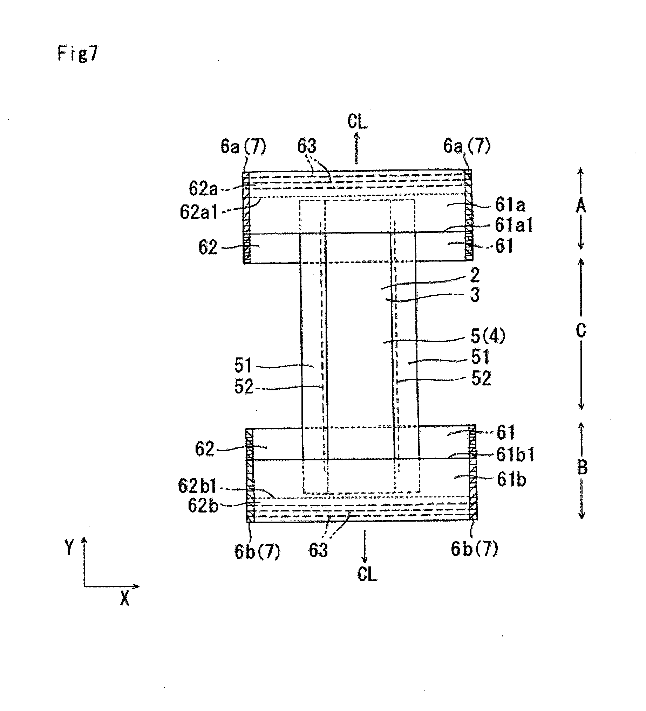

For example, in the pull-on disposable diapers 1A, 1B of the foregoing first and second embodiments, the envelope 6 made up of the outer-layer sheet 61 and the inner-layer sheet 62 extends over the entire outer region of the absorbent body 5, as illustrated in Figs. 2 and 5. However, as illustrated in Fig. 7, the outer-layer sheet 61 and the inner-layer sheet 62 may be disposed only in the area of the stomach-side section A and the area of the back-side section B and the envelope 6 may be formed partially in the stomach-side section A and the back-side section B. Note that the pull-on disposable diaper illustrated in Fig. 7 is a modified embodiment of the pull-on disposable diaper 1A of the first embodiment.

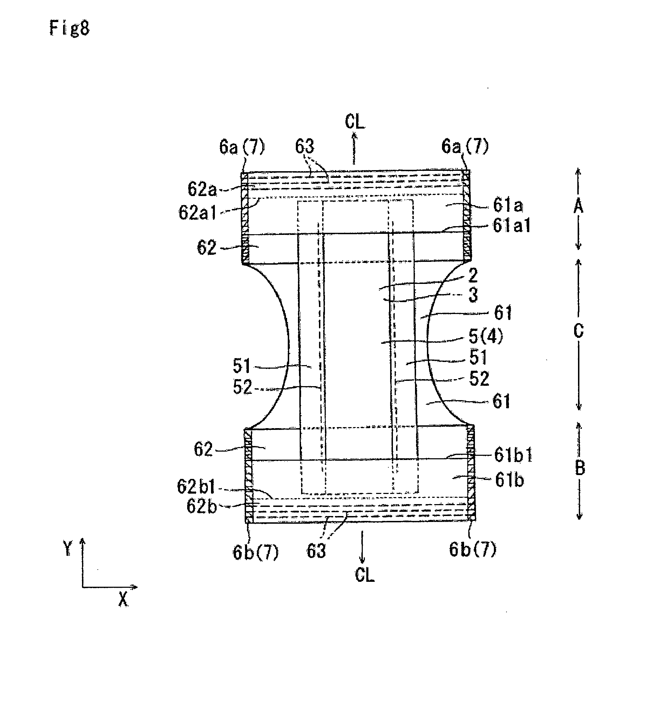

Further, in the pull-on disposable diapers 1A, 1B of the foregoing first and second embodiments, the outer-layer sheet 61 and the inner-layer sheet 62 extend over the entire outer region of the absorbent body 5, as illustrated in Figs. 2 and 5. However, as illustrated in Fig. 8, the envelope 6 may be formed by extending either one of the outer-layer sheet 61 or the inner-layer sheet 62 over the entire outer region of the absorbent body 5. Note that the pull-on disposable diaper illustrated in Fig. 8 is a modified embodiment of the pull-on disposable diaper 1A of the first embodiment, and the envelope 6 is formed by extending the outer-layer sheet 61 over the entire outer region of the absorbent body 5.

Further, in the pull-on disposable diapers 1A, 1B of the foregoing first and second embodiments, the overlapped sections 7 are made up of the envelope 6, as illustrated in Figs. 2 and 5. However, the overlapped sections 7 may be formed of the topsheet 2 and the backsheet 3, without providing an envelope 6, by extending the topsheet 2 and the backsheet 3, which sandwich the absorbent core 4, over the entire outer region of the absorbent core 4.

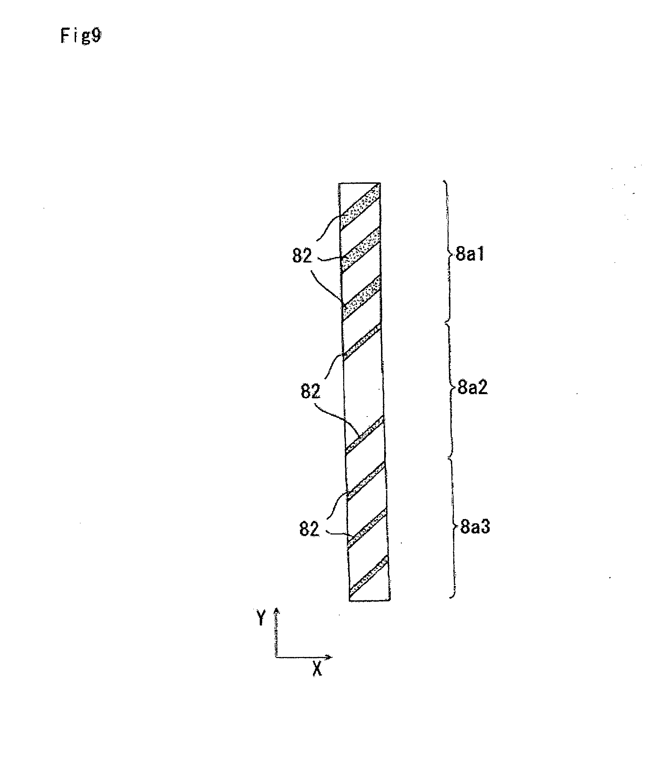

Further, in the pull-on disposable diaper 1A of the foregoing first embodiment, the side-sealing section 8 includes fusion-bonded sections 81 extending in the width direction (X direction) and fusion-bonded sections 82 extending in the oblique direction, as illustrated in Fig. 3(c). However, the fusion-bonded sections 81 do not have to be provided. In such cases, it is preferable that the width of each fusion-bonded section 82 in the sealing region 8a2 is 0.5 mm to 2 mm, the width of each fusion-bonded section 82 in the sealing region 8a3 is 0.5 mm to 3 mm, and the width of each fusion-bonded section 82 in the sealing region 8a1 is 1 mm to 4 mm, as illustrated in Fig. 9. Further, it is preferable that the distance between adjacent fusion-bonded sections 82 in the sealing region 8a2 is 1 mm to 4 mm, the distance between adjacent fusion-bonded sections 82 in the sealing region 8a3 is 1 mm to 3 mm, and the distance between adjacent fusion-bonded sections 82 in the sealing region 8a1 is 0.5 mm to 2 mm. Furthermore, the side-sealing section 8 may include only the fusion-bonded sections 81 extending in the width direction (X direction) and not the obliquely-extending fusion-bonded sections 82.

Further, in the pull-on disposable diaper 1A of the foregoing first embodiment, the side-sealing sections 8 are formed by a single embossing process, as illustrated in Fig. 4. The present invention achieves an advantage that desired side-sealing sections can be formed through a single embossing process by varying the sealing patterns from region to region, without incurring facility loads such as performing embossing a plurality of times. The invention, however, is not limited to the above, and for example, the side-sealing sections 8 may be formed by performing embossing three times using an embossing roll having projections corresponding to the shape of the sealing region 8a1, an embossing roll having projections corresponding to the shape of the sealing region 8a2, and an embossing roll having projections corresponding to the shape of the sealing region 8a3.

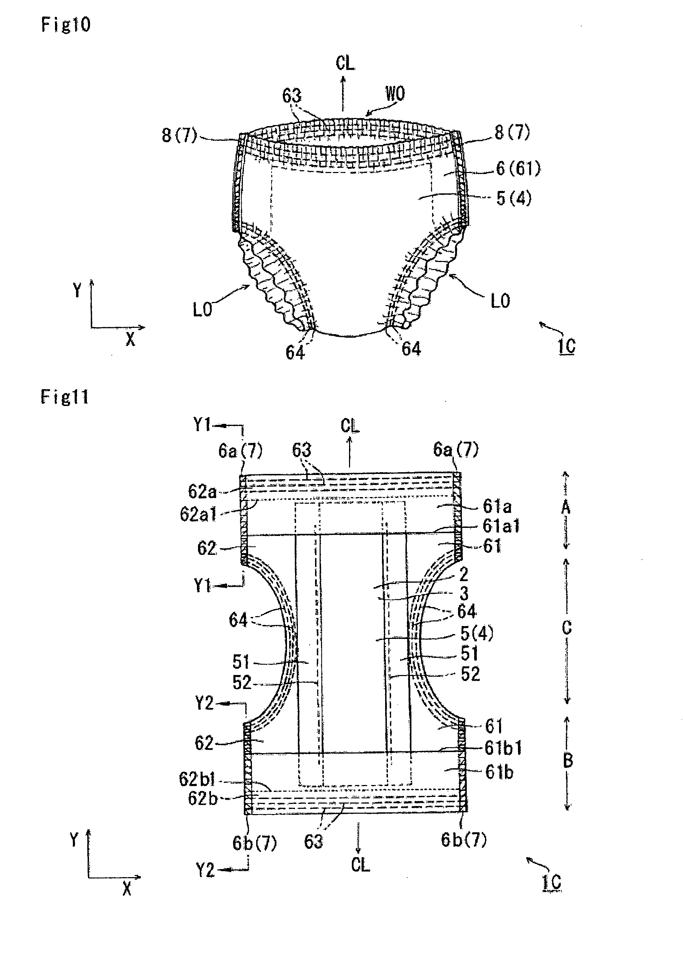

Next, a preferred third embodiment of a pull-on disposable diaper, serving as a pull-on absorbent article of the present invention, is described below with reference to Figs. 10 to 13.

The following describes features of the cleaning sheet 1C of the third embodiment that are different from the pull-on disposal diaper 1A of the foregoing first embodiment. Features that are not particularly explained are the same as those of the cleaning sheet 1A of the first embodiment and explanation on the cleaning sheet 1A of the first embodiment applies as appropriate to such features.

A pull-on disposable diaper 1C of the third embodiment includes a pair of side-sealing sections 8, 8 each being formed by fusion-bonding of respective overlapped sections 7, 7 where opposite side-edge sections 6a, 6a of a stomach-side section A and opposite side-edge sections 6b, 6b of a back-side section B overlap one another. Each of the side-sealing sections 8, 8 includes regions each having a different number of members that make up each overlapped section 7. The larger the number of members that make up the overlapped section 7 is, the larger the total basis weight of the respective basis weights of the plurality of the members becomes in each region. As in the pull-on disposal diaper 1A of the first embodiment, the larger the total basis weight of the respective basis weights of the plurality of the members is, the smaller the fusion-bonded area of the side-sealing section 8 is made. Accordingly, in the pull-on disposable diaper 1C of the third embodiment, the larger the number of members that make up the overlapped section 7 is, the smaller the fusion-bonded area of the side-sealing section 8 is made.

As illustrated in Figs. 10 and 11, the pull-on disposable diaper 1C of the third embodiment is formed to have line symmetry with respect to a center line CL. Accordingly, in the following description, the left-hand side will mainly be described for sections that have line symmetry.

The pull-on disposable diaper 1C of the third embodiment is described in detail. As illustrated in Fig. 11, the pull-on disposable diaper 1C (also referred to hereinafter as "diaper 1C") of the third embodiment includes: a substantially-elongated absorbent body 5 having a liquid-permeable topsheet 2, a liquid-impermeable (or water-repellent) backsheet 3, and an absorbent core 4 provided between the topsheet 2 and the backsheet 3; and an envelope 6 disposed on the backsheet side of the absorbent body 5 and attached and fixed thereto.

As illustrated in Fig. 11, the envelope 6 has a stomach-side section A, a crotch section C, and a back-side section B in its length direction (referred to hereinafter as "Y direction"; the Y direction is a direction parallel to the center line CL). A pair of side-sealing sections 8, 8 is formed by fusion-bonding respective overlapped sections 7, 7 where the opposite side-edge sections 6a, 6a of the stomach-side section A of the envelope 6 and the opposite side-edge sections 6b, 6b of the back-side section B of the envelope 6 overlap one another, thus forming a pull-on disposable diaper 1C having a waist opening WO and a pair of leg openings LO, LO, as illustrated in Fig. 10. The stomach-side section A of the envelope 6 is a section to be located on the wearer's stomach side when the diaper 1C is worn, the back-side section B is a section to be located on the wearer's back side, and the crotch section C is a section to be located in the wearer's crotch.

The envelope 6 extends over the entire outer region of the absorbent body 5, and includes an outer-layer sheet 61 forming an outer surface of the diaper, an inner-layer sheet 62 disposed on the inner side of the outer-layer sheet 61, and folded sections 61a, 61b or 62a, 62b formed by folding, toward the inner-layer sheet 62, outward-extending portions of the outer-layer sheet 61 or the inner-layer sheet 62 that extend outward lengthwise (Y-direction-wise) from the absorbent body 5. As illustrated in Fig. 11, the opposite side edges of the envelope 6 of the present diaper 1C are constricted inward at the lengthwise (Y-direction-wise) central section. Further, as illustrated in Figs. 10 to 12, the envelope 6 is made up of the outer-layer sheet 61 forming an outer surface of the diaper, the inner-layer sheet 62 disposed on the inner side of the outer-layer sheet 61, and a plurality of waist elastic members 63 and a plurality of leg elastic members 64 disposed and fixed between the two sheets 61, 62. The plurality of waist elastic members 63 are provided along the circumferential edge section of the stomach-side section A and the back-side section B which becomes the waist opening WO, and are disposed and fixed along the width direction (referred to hereinafter as "X direction"; the X direction is a direction perpendicular to the center line CL extending in the length direction of the disposable diaper 1C) in their stretched state. The plurality of leg elastic members 64 are disposed along the circumferential edge sections that become the leg openings LO, as illustrated in Fig. 11, and are disposed and fixed in their stretched state.

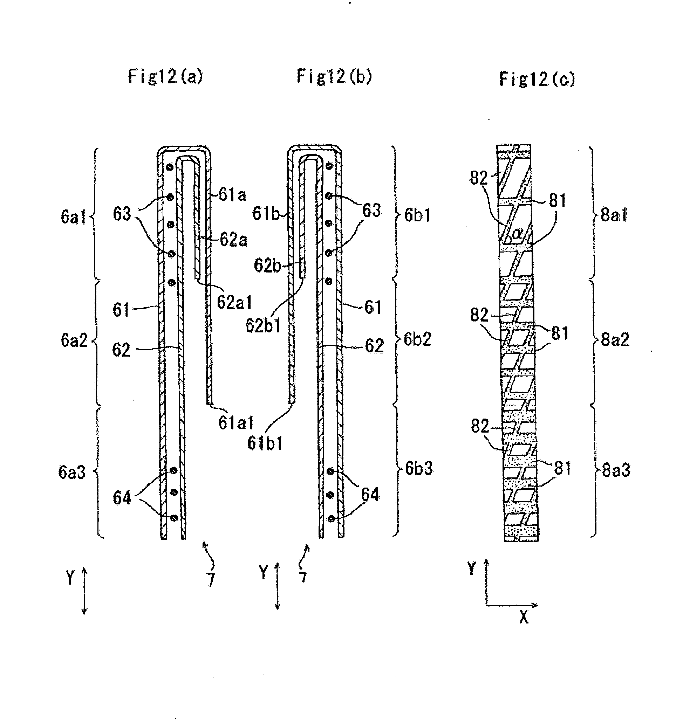

The outer-layer sheet 61 of the envelope 6 of the diaper 1C has, in both the stomach-side section A and the back-side section B, a length that extends further outward lengthwise (Y-direction-wise) from sections where the waist elastic members 63 are sandwiched and fixed between the outer-layer sheet 61 and the inner-layer sheet 62 as illustrated in Fig. 11, Fig. 12(a), and Fig. 12(b). Folding back the extending portions toward the inner-layer sheet 62 will form the folded sections 61a, 61b. As illustrated in Fig. 11 and Fig. 12(a), the lengthwise (Y-direction-wise) end 61a1 of the folded section 61a extends beyond the lengthwise (Y-direction-wise) end of the absorbent body 5 on the side of the stomach-side section A and more toward the crotch section C. Likewise, as illustrated in Fig. 11 and Fig. 12(b), the lengthwise (Y-direction-wise) end 61b1 of the folded section 61b extends beyond the lengthwise (Y-direction-wise) end of the absorbent body 5 on the side of the back-side section B and more toward the crotch section C. In this way, the opposite lengthwise (Y-direction-wise) ends of the absorbent body 5 are respectively sandwiched between the envelope 6 and the respective folded sections 61a, 61b.

The inner-layer sheet 62 of the envelope 6 of the diaper 1C has, in both the stomach-side section A and the back-side section B, a length that extends further outward lengthwise (Y-direction-wise) from sections where the waist elastic members 63 are sandwiched and fixed between the outer-layer sheet 61 and the inner-layer sheet 62 as illustrated in Fig. 11, Fig. 12(a), and Fig. 12(b). Folding back the extending portions toward the inner-layer sheet 62 will form the folded sections 62a, 62b. As illustrated in Fig. 11 and Fig. 12(a), the lengthwise (Y-direction-wise) end 62a1 of the folded section 62a extends up to the vicinity of the lengthwise (Y-direction-wise) end of the absorbent body 5 on the side of the stomach-side section A. Likewise, as illustrated in Fig. 11 and Fig. 12(b), the lengthwise (Y-direction-wise) end 62b1 of the folded section 62b extends up to the vicinity of the lengthwise (Y-direction-wise) end of the absorbent body 5 on the side of the back-side section B. In this way, the lengthwise (Y-direction-wise) length of the folded sections 62a, 62b is made shorter than the lengthwise (Y-direction-wise) length of the folded sections 61a, 61b.

In the present diaper 1C, it is preferable that the basis weight of the inner-layer sheet 62 of the envelope 6 is substantially the same as the basis weight of the outer-layer sheet 61. From the standpoint of texture and cost, it is preferable that in the present diaper 1C, the basis weight of the outer-layer sheet 61 and the basis weight of the inner-layer sheet 62 are 8 g/m² to 30 g/m². The basis weight is measured as follows.

### Method for Measuring Basis Weight

A measurement piece that is 30 mm wide and 30 mm long is cut out from each of the inner-layer sheet 62 and the outer-layer sheet 61, to obtain measurement pieces each having a total area of 900 mm² from a single diaper. The measurement piece is weighed using an electronic balance having a minimum display of 1 mg, and the measured value is converted into basis weight. An average value of measurement pieces sampled from five (n = 5) diapers is regarded as the basis weight value. Note that the measurement pieces of the inner-layer sheet 62 and the outer-layer sheet 61 are to be sampled from sections except for the side-sealing sections 8. In cases where it is not possible to sample a measurement piece that is 30 mm wide and 30 mm long, a piece having the largest possible area should be sampled, and the basis weight can be obtained through conversion based on the area thereof. In cases where the inner-layer sheet and the outer-layer sheet are joined using an adhesive such as a hot-melt adhesive, the weight should be measured after washing off the adhesive with an organic solvent, such as chloroform, and then thoroughly drying the piece. The method above is to be used for measuring the basis weight of members other than the members that form the envelope 6.

As illustrated in Figs. 10 and 11, the overlapped section 7 of the diaper 1C is formed of the envelope 6. More specifically, as illustrated in Fig. 12(a), each side-edge section 6a of the stomach-side section A includes the outer-layer sheet 61, the inner-layer sheet 62, the folded section 61 a, and the folded section 62a, and as illustrated in Fig. 12(b), each side-edge section 6b of the back-side section B includes the outer-layer sheet 61, the inner-layer sheet 62, the folded section 61b, and the folded section 62b. The section where the side-edge section 6a of the stomach-side section A and the side-edge section 6b of the back-side section B overlap one another becomes the overlapped section 7, as illustrated in Fig. 11.

As illustrated in Fig. 12(a), in the present diaper 1C, the side-edge section 6a of the stomach-side section A has, in the length direction (Y direction): a region 6a 1 made up of four members, i.e., the outer-layer sheet 61, the inner-layer sheet 62, the folded section 61a, and the folded section 62a; a region 6a2 made up of three members, i.e., the outer-layer sheet 61, the inner-layer sheet 62, and the folded section 61a; and a region 6a3 made up of two members, i.e., the outer-layer sheet 61 and the inner-layer sheet 62. Likewise, as illustrated in Fig. 12(b), the side-edge section 6b of the black-side section B has, in the length direction (Y direction): a region 6b1 made up of four members, i.e., the outer-layer sheet 61, the inner-layer sheet 62, the folded section 61b, and the folded section 62b; a region 6b2 made up of three members, i.e., the outer-layer sheet 61, the inner-layer sheet 62, and the folded section 61b; and a region 6b3 made up of two members, i.e., the outer-layer sheet 61 and the inner-layer sheet 62. In this way, each overlapped section 7 where the side-edge section 6a and the side-edge section 6b overlap one another includes a region where the region 6a1 and the region 6b1 overlap one another and where the number of members is eight, a region where the region 6a2 and the region 6b2 overlap one another and where the number of members is six, and a region where the region 6a3 and the region 6b3 overlap one another and where the number of members is four. Fusion-bonding this overlapped section 7 will form the side-sealing section 8.

In the side-sealing section 8, a region where the number of members that make up the overlapped section 7 is larger will have a larger total basis weight of the members' respective basis weights, and therefore the fusion-bonded area is made smaller in that region. As illustrated in Fig. 12(c), in the side-sealing section 8 of the diaper 1C, the fusion-bonded area in a sealing region 8a1 formed by fusion-bonding the region where the number of members is eight is made smaller than both the fusion-bonded area in a sealing region 8a2 formed by fusion-bonding the region where the number of members is six and the fusion-bonded area in a sealing region 8a3 formed by fusion-bonding the region where the number of members is four, and further, the fusion-bonded area in the sealing region 8a2 formed by fusion-bonding the region where the number of members is six is made smaller than the fusion-bonded area in the sealing region 8a3 formed by fusion-bonding the region where the number of members is four. More specifically, as illustrated in Fig. 12(c), each of the sealing region 8a1, the sealing region 8a2, and the sealing region 8a3 includes fusion-bonded sections 81 extending along the width direction (X direction) and fusion-bonded sections 82 extending obliquely toward the center of the diaper 1C's lengthwise (Y-direction-wise) end on the side of the stomach-side section A. The width of each fusion-bonded section 81 in the sealing region 8a1 is made narrower than the width of each fusion-bonded section 81 in both the sealing region 8a2 and the sealing region 8a3, and further, the width of each fusion-bonded section 81 in the sealing region 8a2 is made narrower than the width of each fusion-bonded section 81 in the sealing region 8a3. The width of the fusion-bonded section 82 is substantially the same in all of the sealing region 8a1, the sealing region 8a2, and the sealing region 8a3, and the distance between adjacent fusion-bonded sections 82 is also substantially the same.

The width of each fusion-bonded section 81 in the sealing region 8a1 is preferably 0.2 mm to 2 mm, the width of each fusion-bonded section 81 in the sealing region 8a2 is preferably 0.4 mm to 2.5 mm, and the width of each fusion-bonded section 81 in the sealing region 8a3 is preferably 0.6 mm to 3 mm. The distance between adjacent fusion-bonded sections 81 in the sealing region 8a1 is preferably 3 mm to 8 mm, the distance between adjacent fusion-bonded sections 81 in the sealing region 8a2 is preferably 2 mm to 7 mm, and the distance between adjacent fusion-bonded sections 81 in the sealing region 8a3 is preferably 1 mm to 6 mm. The angle α illustrated in Fig. 12(c) at which the fusion-bonded section 81 intersects with the fusion-bonded section 82 is preferably 30° to 70°. The width of each fusion-bonded section 82 in the sealing region 8a1, the sealing region 8a2, and the sealing region 8a3 is preferably 0.5 mm to 3 mm, and the distance between adjacent fusion-bonded sections 82 is preferably 1 mm to 5 mm.

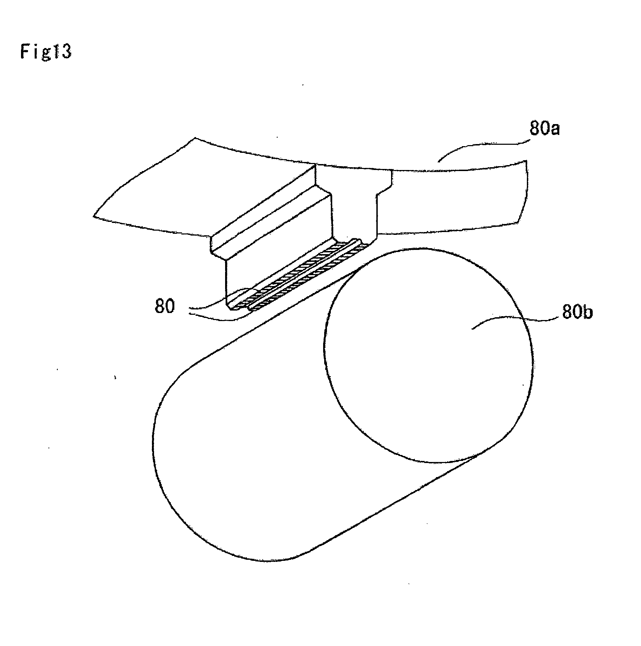

Fusion-bonding of the side-sealing section 8 is achieved by employing, for example, heat embossing, ultrasonic embossing, or high-frequency embossing. In the present diaper 1C, a pair of ultrasonic embossing rolls 80a, 80b is employed, as illustrated in Fig. 13. More specifically, a continuous strip of diapers 1C is passed between an embossing roll 80a and a flat roll 80b, wherein the embossing roll 80a has projections 80 shaped so as to correspond to the shape of the side-sealing sections 8 illustrated in Fig. 12(c), and the flat roll 80b has a flat surface. The projections 80 and the flat roll 80b perform embossing at the position of the overlapped section 7 where the side-edge section 6a and the side-edge section 6b overlap one another, to thus form a side-sealing section 8. Each projection 80 includes projecting pieces that correspond to the respective shapes of the sealing region 8a1, the sealing region 8a2, and the sealing region 8a3, wherein the projecting pieces are arranged in a direction orthogonal to the flow direction of the continuous strip of diapers 1C such that the direction orthogonal to the flow direction matches the length direction (Y direction) of the side-sealing section 8 illustrated in Fig. 12(c). Accordingly, the side-sealing section 8 is formed by a single embossing process.

Herein, the "fusion-bonded area of the sealing region 8a1", the "fusion-bonded area of the sealing region 8a2", and the "fusion-bonded area of the sealing region 8a3" each refer to the area of portions embossed by the projection 80, and more specifically, refer to the bottom-surface area of embossed portions that have been turned into a film by the embossing process when viewing the side-sealing section 8 from above. Note that in cases where the side-sealing section 8 has shrunk in the width direction (X direction) etc. by means of the waist elastic members 63 etc., the portion's bottom-surface area is to be measured in its stretched-out state.

It is preferable that the fusion-bond strength of the side-sealing section 8 is 5 N/30 mm to 40 N/30 mm, and more preferably 5 N/30 mm to 30 N/30 mm. The "fusion-bond strength of the side-sealing section 8" means that the fusion-bond strength in each of the sealing region 8a1, the sealing region 8a2, and the sealing region 8a3 falls within the above numerical range. Setting the fusion-bond strength of the side-sealing section 8 within the numerical range of 5 N/30 mm to 40 N/30 mm will prevent the side-sealing sections 8 from peeling apart during use, but will allow the side-sealing sections 8 to be torn easily after use to thus facilitate diaper change.

It is preferable that a ratio (Fmin/Fmax) of the minimum fusion-bond strength of the side-sealing section 8 to the maximum fusion-bond strength of the side-sealing section is 2/3 < Fmin/Fmax ≤ 1, and more preferably 5/6 < Fmin/Fmax ≤ 1. Setting the ratio (Fmin/Fmax) of the minimum fusion-bond strength of the side-sealing section 8 to the maximum fusion-bond strength of the side-sealing section within the numerical range of 2/3 < Fmin/Fmax ≤ 1 will allow the side-sealing sections 8 to be torn easily after use with uniform force because of the small variation in fusion-bond strength, thus further facilitating diaper change. The fusion-bond strength is measured as follows.

### Method for Measuring Fusion-bond Strength

The side-sealing section 8 is cut out from a diaper 1C along the length direction (Y direction) thereof. Next, the cut-out side-sealing section 8 is cut widthwise (X-direction-wise) in the sealing region 8a1, the sealing region 8a2, and the sealing region 8a3 to obtain a sample of the sealing region 8a1, a sample of the sealing region 8a2, and a sample of the sealing region 8a3, the lengthwise (Y-direction-wise) length of the sealing section of each sample being 30 mm.

The sealing strength in each region is measured using a tensile tester (Autograph AGS50A (trade name) manufactured by Shimadzu Corporation). Measurement is to be performed at a tensile speed of 300 mm/min to determine the maximum strength. Measurement is to be performed five (n = 5) times, and an average value thereof is regarded as the fusion-bond strength for each region. In cases where it is not possible to cut out a sample with a sealing-section length of 30 mm from each region, the fusion-bond strength can be obtained by performing measurement with the largest-possible length closest to 30 mm and converting the measurement value to a value for the 30-mm length.

As illustrated in Fig. 11, the absorbent body 5 is substantially elongated and has a liquid-permeable topsheet 2, a liquid-impermeable (or water-repellent) backsheet 3, and a liquid-retainable absorbent core 4 provided between the topsheet 2 and the backsheet 3. As illustrated in Fig. 11, the absorbent body 5 is disposed from the back-side section B of the envelope 6 to the stomach-side section A thereof, and the opposite lengthwise (Y-direction-wise) ends of the absorbent body 5 are located at positions setting back inward in the length direction (Y direction) from the respective opposite lengthwise (Y-direction-wise) ends of the envelope 6. The absorbent body 5 is joined to the inner-layer sheet 62 of the envelope 6 through joining such as by heat sealing or ultrasonic sealing, with an adhesive, and the like.

As illustrated in Fig. 11, the opposite lengthwise (Y-direction-wise) side sections of the absorbent body 5 have side cuffs 51, 51 made of a liquid-resistible/water-repellent and breathable material. An elastic member 52 for forming the side cuff is disposed in its stretched state along the length direction (Y direction) in the vicinity of the free end of each side cuff 51 and fixed thereto. When the diaper is worn, the respective free-end sides of the side cuffs 51 stand up, thus preventing excrement from flowing out from the absorbent body 5 in the width direction (X direction). Each sheet for forming the side cuff 51 is fixed such that a portion thereof located on the outer side, in the width direction (X direction) of the absorbent body 5, and having a predetermined width is tucked in toward the skin-non-contacting side.

Materials for forming the pull-on disposable diaper 1C of the third embodiment are described below.

It is preferable that the fusion-bonding component of the members that make up the overlapped section 7 is made of a general-purpose resin, such as polypropylene, polyethylene, or polyester, in terms of cost, and it is preferable that the respective fusion-bonding components of the members that make up the overlapped section 7 are components of the same series. In the present diaper 1C, the members that make up the overlapped section 7 are the outer-layer sheet 61 (including the folded section 61a) and the inner-layer sheet 62 (including the folded section 62a). Accordingly, it is preferable that the outer-layer sheet 61 and the inner-layer sheet 62 are of components of the same series. "Components of the same series as polypropylene" serving as respective fusion-bonding components of the outer-layer sheet 61 and the inner-layer sheet 62 include, for example: nonwoven sheets made only of homo-PP fibers containing polypropylene monomers; nonwoven sheets made only of random-PP fibers in which a small amount of ethylene etc. is introduced randomly into the propylene chain; nonwoven sheets made of sheath/core conjugate fibers or side-by-side conjugate fibers that contain 50% or more of homo-PP resin or random-PP resin and also contain thermoplastic resin other than homo-PP resin or random-PP resin (such as polyethylene resin, polyester resin, etc.); and nonwoven sheets made of fibers that contain 50% or more of homo-PP fibers or random-PP fibers and also contain fibers other than homo-PP fibers or random-PP fibers (such as rayon fiber, cotton fiber, polyethylene fiber, polyester fiber, etc.).

The phrase "respective fusion-bonding components of the outer-layer sheet 61 and the inner-layer sheet 62 are components of the same series as polyethylene or polyester" can be construed similarly as the "components of the same series as polypropylene" . Particularly, "components of the same series as polyethylene" preferably include, for example, nonwoven sheets made of fibers admixed with sheath/core conjugate fibers or side-by-side conjugate fibers containing polyester resin as the core and polyethylene resin as the sheath and/or admixed with sheath/core conjugate fibers or side-by-side conjugate fibers containing polypropylene resin as the core and polyethylene resin as the sheath. Further, particularly, "components of the same series as polyester" preferably include, for example, nonwoven sheets made of fibers admixed with sheath/core conjugate fibers or side-by-side conjugate fibers containing polyester resin as the core and low-melting-point polyester resin as the sheath. Composing the respective fusion-bonding components of the members that make up the overlapped section 7 out of components of the same series as polypropylene, polyethylene, or polyester provides good compatibility among the fusion-bonding components as well as favorable fusion-bond strength, thereby allowing fusion-bonding to be performed at lower temperatures. Because embossing does not have to be performed at high temperatures, portions other than the embossed portions will not stiffen, thus making the side-sealing sections soft as a whole and improving the feel against the skin.

Any kind of material generally used for absorbent articles, such as disposable diapers, can be used without particular limitation for the topsheet 2, the backsheet 3, and the sheets for forming the side cuffs 51. For the topsheet 2, it is possible to use, for example, a liquid-permeable nonwoven fabric, a porous film, or a laminate thereof. For the backsheet 3, it is possible to use, for example, a resin film or a laminate of a resin film and a nonwoven fabric. For the sheets for forming the side cuffs 51, it is possible to use, for example, stretchable films, nonwoven fabrics, woven fabrics, or laminated sheets made of the above.

Any kind of material generally used for absorbent articles, such as disposable diapers, can be used without particular limitation for the absorbent core 4. For example, for the absorbent core 4, it is possible to use either a fiber aggregate made of fibrous material such as pulp and wrapped with a covering such as tissue paper or a permeable nonwoven fabric, or the above fiber aggregate further containing high-absorbent polymer particles and wrapped with a like covering.

Any kind of material generally used for absorbent articles, such as disposable diapers, can be used without particular limitation for the elastic members 52 for forming the side cuffs, the waist elastic members 63, and the leg elastic members 64. For example, it is possible to use stretchable materials made of natural rubber, polyurethane, polystyrene-polyisoprene copolymer, polystyrene-polybutadiene copolymer, or polyethylene-α-olefin copolymer such as ethyl acrylate-ethylene.

The following describes effects achieved by using the pull-on disposable diaper 1C of the foregoing third embodiment of the present invention.

As illustrated in Figs. 10 to 12, in the diaper 1C of the third embodiment, the larger the number of members that make up each of the overlapped sections 7, 7 is, the smaller the fusion-bonded area of the side-sealing section 8 is made. In general, as the number of members forming the overlapped section 7 increases, the amount of fusion-bonded resin also increases along therewith. Therefore, the portion with the increased resin amount becomes stiffer when that portion is thermally resinified. Such stiff portions may hurt the skin or cause other problems when the article is worn. In contrast, in the present invention, the fusion-bonded area is varied in accordance with the number of members and the total fusion-bonded area of the entire side-sealing section 8 can thus be kept within certain bounds, even in cases where the number of members that make up the overlapped sections 7, 7--where the opposite side-edge sections 6a, 6a of the stomach-side section A and the opposite side-edge sections 6b, 6b of the back-side section B overlap one another--becomes partially large and the amount of resin that constitutes the fusion-bonding components also becomes large, as described above. Thus, the side-sealing sections 8 are less prone to become stiff and thus less prone to cause pain when the absorbent article is worn or to hurt the skin. Furthermore, the fusion-bond strength in each region becomes substantially the same. Thus, when the side sections are to be peeled apart after use, the peeling can be done with a uniform force, thereby improving usability.

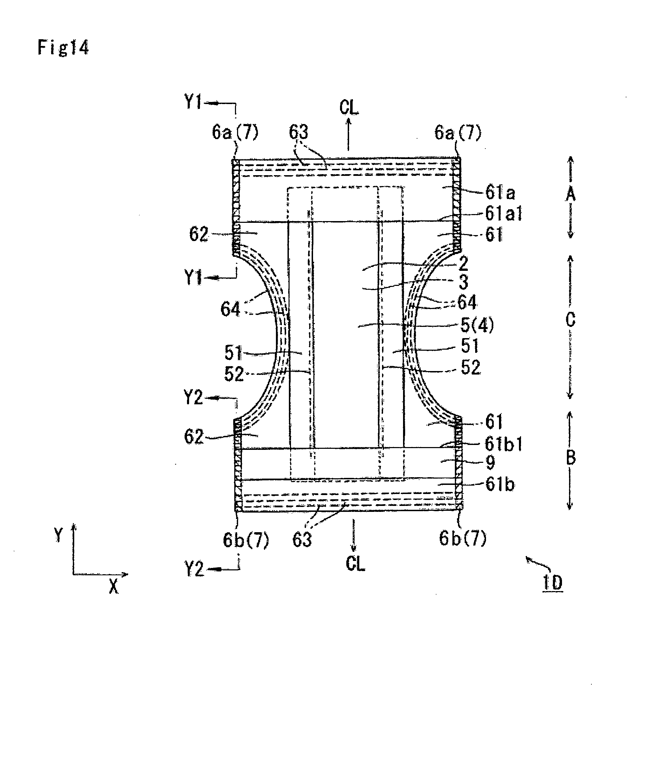

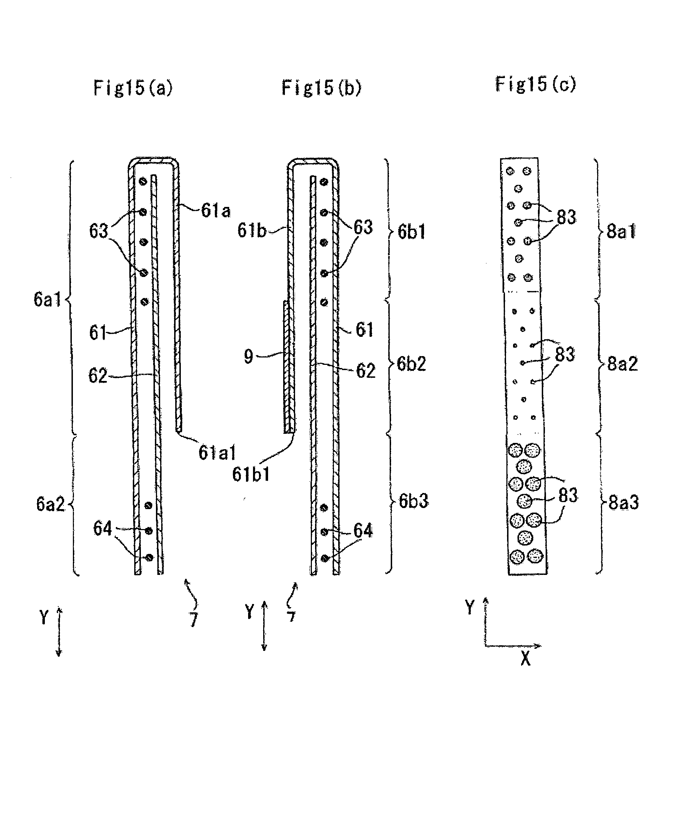

Next, a pull-on disposable diaper according to a fourth embodiment of the pull-on absorbent article of the present invention is described below with reference to Figs. 14 and 15.

The following mainly describes features of the pull-on disposable diaper 1D of the fourth embodiment that are different from the pull-on disposable diaper 1C of the foregoing third embodiment, and features in common are accompanied with the same symbols and are omitted from explanation. The explanation on the pull-on disposable diaper 1C of the third embodiment applies as appropriate to features that are not particularly explained below.

In the pull-on disposable diaper 1D (also referred to hereinafter as "diaper 1D") of the fourth embodiment, the overlapped section 7 is configured to include a member, such as a sweat-absorbent sheet, besides the members forming the envelope 6. More specifically, as illustrated in Figs. 14 and 15, the diaper 1D differs greatly from the diaper 1C in that it has a sweat-absorbent sheet 9 extending in the width direction (X direction) on the back-side section B. The diaper 1D is described in detail below.

As illustrated in Fig. 14, the envelope 6 of the diaper 1D extends over the entire outer region of the absorbent body 5, and includes an outer-layer sheet 61 forming an outer surface of the diaper, an inner-layer sheet 62 disposed on the inner side of the outer-layer sheet 61, and folded sections 61a, 61b formed by folding, toward the inner-layer sheet 62, outward-extending portions of the outer-layer sheet 61 that extend outward lengthwise (Y-direction-wise) from the absorbent body 5. The outer-layer sheet 61 of the envelope 6 of the diaper 1D has, in both the stomach-side section A and the back-side section B, a length that extends further outward lengthwise (Y-direction-wise) from sections where the waist elastic members 63 are sandwiched and fixed between the outer-layer sheet 61 and the inner-layer sheet 62 as illustrated in Figs. 14 and 15. Folding back the extending portions toward the inner-layer sheet 62 will form the folded sections 6 1 a, 61b. As illustrated in Fig. 14 and Fig. 15(a), the lengthwise (Y-direction-wise) end 61a1 of the folded section 61a extends beyond the lengthwise (Y-direction-wise) end of the absorbent body 5 on the side of the stomach-side section A and more toward the crotch section C. Likewise, as illustrated in Fig. 14 and Fig. 15(b), the lengthwise (Y-direction-wise) end 61b1 of the folded section 61b extends beyond the lengthwise (Y-direction-wise) end of the absorbent body 5 on the side of the back-side section B and more toward the crotch section C. In this way, the opposite lengthwise (Y-direction-wise) ends of the absorbent body 5 are respectively sandwiched between the envelope 6 and the respective folded sections 61a, 61b.

As illustrated in Fig. 14, the inner-layer sheet 62 of the envelope 6 of the diaper 1D extends, in both the stomach-side section A and the back-side section B, from the waist elastic members 63 of the stomach-side section A to the waist elastic members 63 of the back-side section B, and thus the waist elastic members 63 are sandwiched and fixed between the inner-layer sheet 62 and the outer-layer sheet 61. In other words, different from the inner-layer sheet 62 of the diaper 1C, the inner-layer sheet 62 of the diaper 1D does not have folded sections 62a, 62b, as illustrated in Fig. 15(a) and Fig. 15(b).

As illustrated in Fig. 14 and Fig. 15(b), the sweat-absorbent sheet 9 of the diaper 1D is disposed extending in the width direction (X direction) on the back-side section B. The sweat-absorbent sheet 9 is disposed on the folded section 61b of the outer-layer sheet 61 and spans the opposite side-edge sections 6b, 6b of the back-side section B. The lengthwise (Y-direction-wise) length of the sweat-absorbent sheet 9 extends from a position near the absorbent body 5's lengthwise (Y-direction-wise) end on the side of the back-side section B to a position substantially matching the folded section 61b's lengthwise (Y-direction-wise) end 61b1.

As illustrated in Fig. 15(a), each side-edge section 6a of the stomach-side section A of the diaper 1D includes the outer-layer sheet 61, the inner-layer sheet 62, and the folded section 61a, and as illustrated in Fig. 15(b), each side-edge section 6b of the back-side section B includes the outer-layer sheet 61, the inner-layer sheet 62, the folded section 61b, and the sweat-absorbent sheet 9.

As illustrated in Fig. 15(a), in the present diaper 1D, the side-edge section 6a of the stomach-side section A has, in the length direction (Y direction): a region 6a1 made up of three members, i.e., the outer-layer sheet 61, the inner-layer sheet 62, and the folded section 61a; and a region 6a2 made up of two members, i.e., the outer-layer sheet 61 and the inner-layer sheet 62. As illustrated in Fig. 15(b), the side-edge section 6b of the black-side section B has, in the length direction (Y direction): a region 6b1 made up of three members, i.e., the outer-layer sheet 61, inner-layer sheet 62, and the folded section 61b; a region 6b2 made up of four members, i.e., the outer-layer sheet 61, the inner-layer sheet 62, the folded section 61b, and the sweat-absorbent sheet 9; and a region 6b3 made up of two members, i.e., the outer-layer sheet 61 and the inner-layer sheet 62. In this way, each overlapped section 7 where the side-edge section 6a and the side-edge section 6b overlap one another includes a region where the region 6a1 and the region 6b1 overlap one another and where the number of members is six, a region where the region 6a1 and the region 6b2 overlap one another and where the number of members is seven, and a region where the region 6a2 and the region 6b3 overlap one another and where the number of members is four.

In the side-sealing section 8 formed by fusion-bonding the overlapped section 7, a region where the number of members that make up the overlapped section 7 is larger will have a larger total basis weight of the members' respective basis weights, and therefore the fusion-bonded area is made smaller in that region. As illustrated in Fig. 15(c), in the side-sealing section 8 of the diaper 1D, the fusion-bonded area in a sealing region 8a2 formed by fusion-bonding the region where the number of members is seven is made smaller than both the fusion-bonded area in a sealing region 8a1 formed by fusion-bonding the region where the number of members is six and the fusion-bonded area in a sealing region 8a3 formed by fusion-bonding the region where the number of members is four, and further, the fusion-bonded area in the sealing region 8a1 formed by fusion-bonding the region where the number of members is six is made smaller than the fusion-bonded area in the sealing region 8a3 formed by fusion-bonding the region where the number of members is four. More specifically, as illustrated in Fig. 15(c), each of the sealing region 8a2, the sealing region 8a1, and the sealing region 8a3 includes a plurality of circular fusion-bonded sections 83. The diameter of each fusion-bonded section 83 in the sealing region 8a2 is made shorter than the diameter of each fusion-bonded section 83 in the sealing region 8a1 and the sealing region 8a3, and the diameter of each fusion-bonded section 83 in the sealing region 8a1 is made shorter than the diameter of each fusion-bonded section 83 in the sealing region 8a3.

The diameter of each fusion-bonded section 83 in the sealing region 8a2 is preferably 1 mm to 3 mm. The diameter of each fusion-bonded section 83 in the sealing region 8a1 is preferably 2 mm to 4 mm. The diameter of each fusion-bonded section 83 in the sealing region 8a3 is preferably 3 mm to 5 mm.

Materials for forming the pull-on disposable diaper 1D of the fourth embodiment are described below.

Only features of the pull-on disposable diaper 1D of the fourth embodiment that are different from those of the pull-on disposable diaper 1C of the third embodiment are described below. Features that are not specifically described are the same as those in the pull-on disposable diaper 1C of the third embodiment.

Any kind of material generally used for absorbent articles, such as disposable diapers, can be used without particular limitation for the sweat-absorbent sheet 9. For example, for the sweat-absorbent sheet 9, it is possible to use a spunlace nonwoven fabric, an air-through nonwoven fabric, paper, or the like that contains at least 50% by weight, and more preferably at least 60% by weight, of hydrophilic fibers such as rayon, cotton, or pulp. Note that in the present diaper 1D, it is preferable that the basis weight of the sweat-absorbent sheet 9 is substantially the same as those of the outer-layer sheet 61 and the inner-layer sheet 62 of the envelope 6. From the standpoint of sweat absorbency, it is preferable that the basis weight of the sweat-absorbent sheet 9 is 8 g/m² to 30 g/m². The basis weight is measured according to the method described further above.

The following describes effects achieved by using the pull-on disposable diaper 1D of the foregoing fourth embodiment of the present invention.

The pull-on disposable diaper 1D of the fourth embodiment achieves similar effects as those of the pull-on disposable diaper 1C of the third embodiment. The following describes effects thereof that differ from those of the pull-on disposable diaper 1C of the third embodiment.

As illustrated in Figs. 14 and 15, the disposable diaper 1D of the fourth embodiment has, in particular, a sweat-absorbent sheet 9 provided on the back-side section B. Therefore, the number of members that make up each of the overlapped sections 7, 7--where the opposite side-edge sections 6a, 6a of the stomach-side section A and the opposite side-edge sections 6b, 6b of the back-side section B overlap one another--becomes partially large. However, since the fusion-bonded area is varied in accordance with the number of members, the total fusion-bonded area of the entire side-sealing section 8 can be kept within certain bounds. Thus, the side-sealing sections 8 are less prone to become stiff and thus less prone to cause pain when the absorbent article is worn or to hurt the skin.

The pull-on absorbent article of the present invention is not limited whatsoever to the pull-on disposable diapers 1C, 1D of the foregoing third and fourth embodiments, but can be modified as appropriate. Further, the constituent features of the pull-on disposable diapers 1C, 1D of the foregoing third and fourth embodiments can be worked in combination as appropriate, as long as such combined use does not contradict the gist of the invention.

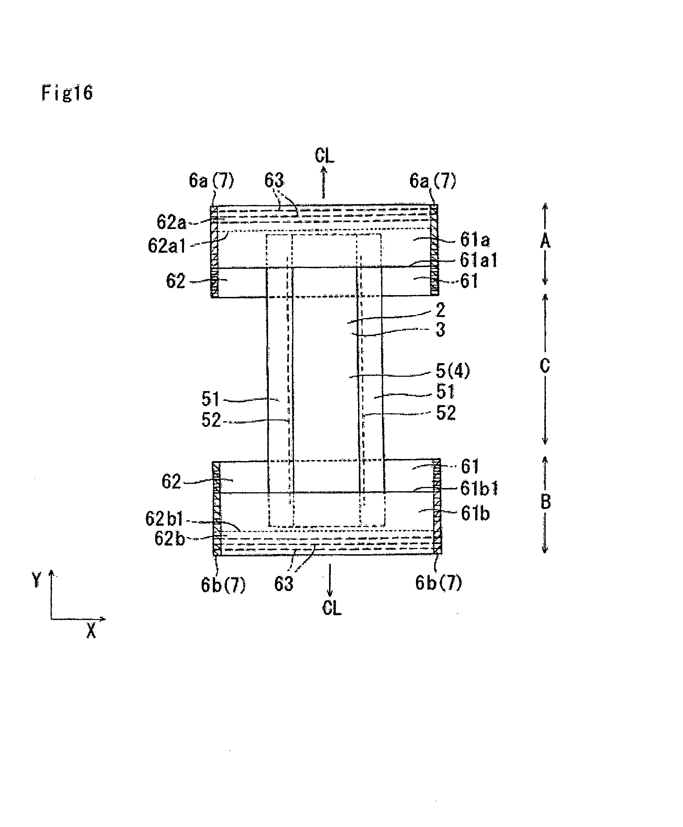

For example, in the pull-on disposable diapers 1C, 1D of the foregoing third and fourth embodiments, the envelope 6 made up of the outer-layer sheet 61 and the inner-layer sheet 62 extends over the entire outer region of the absorbent body 5, as illustrated in Figs. 11 and 14. However, as illustrated in Fig. 16, the outer-layer sheet 61 and the inner-layer sheet 62 may be disposed only in the area of the stomach-side section A and the area of the back-side section B and the envelope 6 may be formed partially in the stomach-side section A and the back-side section B. Note that the pull-on disposable diaper illustrated in Fig. 16 is a modified embodiment of the pull-on disposable diaper 1C of the third embodiment.

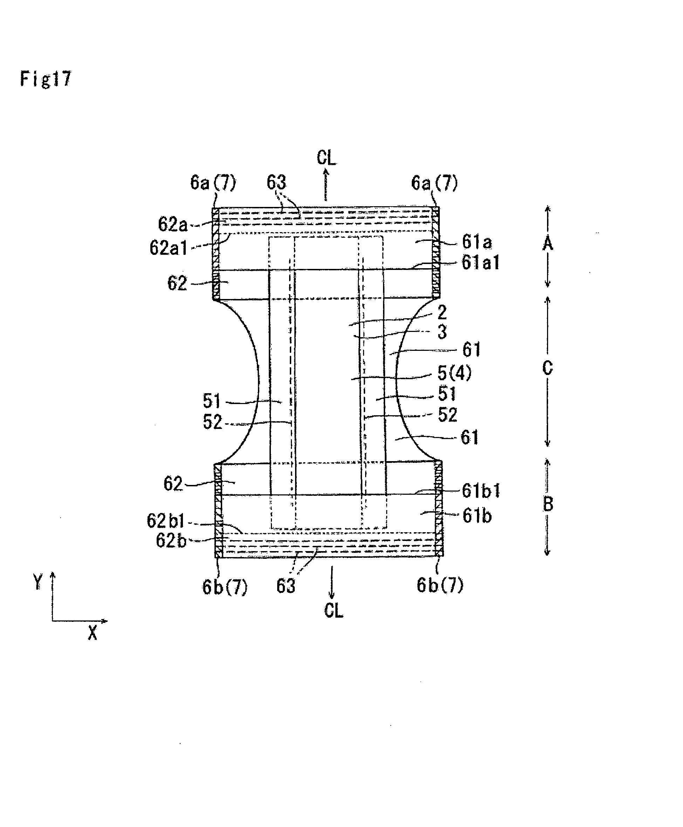

Further, in the pull-on disposable diapers 1C, 1D of the foregoing third and fourth embodiments, the outer-layer sheet 61 and the inner-layer sheet 62 extend over the entire outer region of the absorbent body 5, as illustrated in Figs. 11 and 14. However, as illustrated in Fig. 17, the envelope 6 may be formed by extending either one of the outer-layer sheet 61 or the inner-layer sheet 62 over the entire outer region of the absorbent body 5. Note that the pull-on disposable diaper illustrated in Fig. 17 is a modified embodiment of the pull-on disposable diaper 1C of the third embodiment, and the envelope 6 is formed by extending the outer-layer sheet 61 over the entire outer region of the absorbent body 5.

Further, in the pull-on disposable diapers 1C, 1D of the foregoing third and fourth embodiments, the overlapped sections 7 are made up of the envelope 6, as illustrated in Figs. 11 and 14. However, the overlapped sections 7 may be formed of the topsheet 2 and the backsheet 3, without providing an envelope 6, by extending the topsheet 2 and the backsheet 3, which sandwich the absorbent core 4, over the entire outer region of the absorbent core 4.

Further, in the pull-on disposable diapers 1C,1D of the foregoing third and fourth embodiments, the outer-layer sheet 61 and/or the inner-layer sheet 62 are formed so that the folded sections 61a, 61b and/or 62a, 62b are symmetrical in the stomach-side section A and the back-side section B, as illustrated in Figs. 11 and 14. However, the folded sections do not have to be symmetrical in the stomach-side section A and the back-side section B.

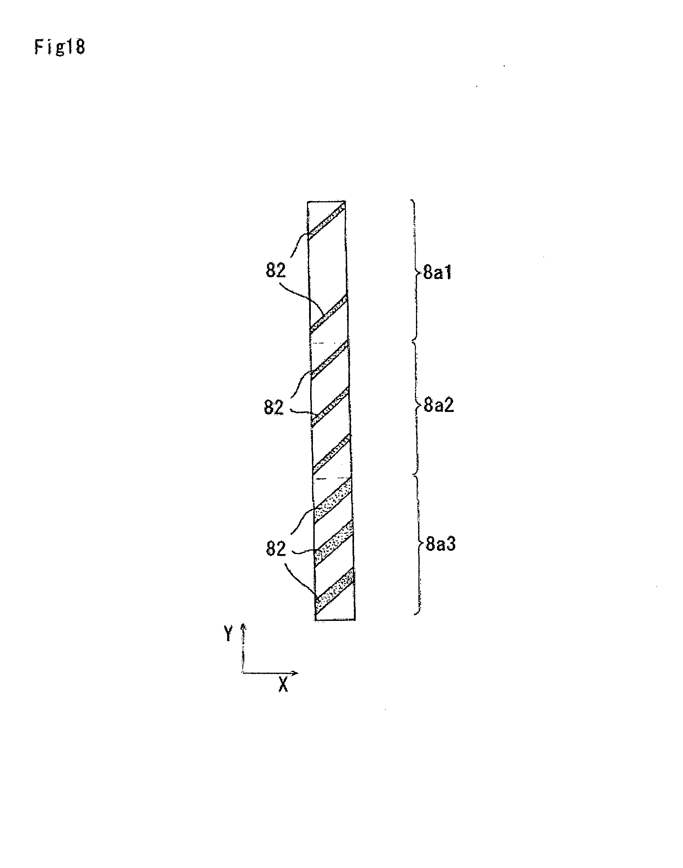

Further, in the pull-on disposable diaper 1C of the foregoing third embodiment, the side-sealing section 8 includes fusion-bonded sections 81 extending in the width direction (X direction) and fusion-bonded sections 82 extending in the oblique direction, as illustrated in Fig. 12(c). However, the fusion-bonded sections 81 do not have to be provided. In such cases, it is preferable that the width of each fusion-bonded section 82 in the sealing region 8a1 is 0.5 mm to 2 mm, the width of each fusion-bonded section 82 in the sealing region 8a2 is 0.5 mm to 3 mm, and the width of each fusion-bonded section 82 in the sealing region 8a3 is 1 mm to 4 mm, as illustrated in Fig. 18. Further, it is preferable that the distance between adjacent fusion-bonded sections 82 in the sealing region 8a1 is 1 mm to 4 mm, the distance between adjacent fusion-bonded sections 82 in the sealing region 8a2 is 1 mm to 3 mm, and the distance between adjacent fusion-bonded sections 82 in the sealing region 8a3 is 0.5 mm to 2 mm. Furthermore, the side-sealing section 8 may include only the fusion-bonded sections 81 extending in the width direction (X direction) and not the obliquely-extending fusion-bonded sections 82.

Further, in the pull-on disposable diaper 1D of the foregoing fourth embodiment, a sweat-absorbent sheet 9 is provided extending in the width direction (X direction) on the back-side section B, as illustrated in Fig. 15(b). However, the sweat-absorbent sheet 9 does not have to be provided, as illustrated in 19(b). In such cases, the overlapped section 7 where the side-edge section 6a and the side-edge section 6b overlap one another is made up of two regions--i.e., a region where the number of members is six and a region where the number of members is four--and the side-sealing section 8 is formed so that the fusion-bonded area of the sealing region formed by fusion-bonding the region where the number of members is six becomes smaller than the fusion-bonded area of the sealing region formed by fusion-bonding the region where the number of members is four.

Further, in the pull-on disposable diaper 1C of the foregoing third embodiment, the side-sealing sections 8 are formed by a single embossing process, as illustrated in Fig. 13. The present invention achieves an advantage that desired side-sealing sections can be formed through a single embossing process by varying the sealing patterns from region to region, without incurring facility loads such as performing embossing a plurality of times. The invention, however, is not limited to the above, and for example, the side-sealing sections 8 may be formed by performing embossing three times using an embossing roll having projections corresponding to the shape of the sealing region 8a1, an embossing roll having projections corresponding to the shape of the sealing region 8a2, and an embossing roll having projections corresponding to the shape of the sealing region 8a3.

Besides pull-on disposable diapers, the present invention can suitably be used for absorbent articles such as pull-on sanitary napkins.

### Industrial Applicability

According to the pull-on absorbent article of the present invention, the side-sealing sections are less prone to become stiff and thus less prone to cause pain when the absorbent article is worn or to hurt the skin, even in cases where the basis weight of the overlapped sections where the opposite side-edge sections of the stomach-side section and the opposite side-edge sections of the back-side section overlap one another becomes partially large.

## Claims

1. A pull-on absorbent article comprising a pair of side-sealing sections each being formed by fusion-bonding of respective overlapped sections where opposite side-edge sections of a stomach-side section and opposite side-edge sections of a back-side section overlap one another, each side-sealing section including regions each having a different total basis weight of members that make up the overlapped section, wherein:
each of the overlapped sections is made up of a plurality of members having respective basis weights; and
the larger a total basis weight of the respective basis weights of the plurality of the members is in a region of the side-sealing section, the smaller a fusion-bonded area is made in that region of the side-sealing section.

2. The pull-on absorbent article according to claim 1, wherein:
the pull-on absorbent article includes: a substantially-elongated absorbent body having a topsheet, a backsheet, and an absorbent core provided between the topsheet and the backsheet; and an envelope disposed on the backsheet side of the absorbent body;
the envelope includes an outer-layer sheet forming an outer surface of the pull-on absorbent article, and an inner-layer sheet disposed on an inner side of the outer-layer sheet; and
the overlapped sections are made of the envelope.

3. The pull-on absorbent article according to claim 2, wherein the envelope has a folded section formed by folding, toward the inner-layer sheet, an outward-extending portion of either the outer-layer sheet or the inner-layer sheet that extends outward lengthwise from the absorbent body.

4. The pull-on absorbent article according to claim 2 or 3, wherein the overlapped section is configured to include a member besides members forming the envelope.

5. The pull-on absorbent article according to claim 1, wherein:
said regions in each side-sealing section respectively having different total basis weights are formed by varying a number of said members that make up the overlapped section; and
the larger the number of said members that make up the overlapped section is, the larger the total basis weight of the respective basis weights of the plurality of the members becomes in each region.

6. The pull-on absorbent article according to any one of claims 1 to 5, wherein a fusion-bond strength of the side-sealing section is 5 N/30 mm to 40 N/30 mm being measured with the method as described therein.

7. The pull-on absorbent article according to any one of claims 1 to 6, wherein a ratio (Fmin/Fmax) of a minimum fusion-bond strength of the side-sealing section to a maximum fusion-bond strength of the side-sealing section is 2/3 < Fmin/Fmax ≤ 1, being measured with the method as described therein.

8. The pull-on absorbent article according to any one of claims 1 to 7, wherein respective fusion-bonding components of said members that make up the overlapped section are components of a same series as polypropylene, polyethylene, or polyester.

## Patentansprüche

1. Anziehbarer absorbierender Artikel mit einem Paar von seitenabdichtenden Abschnitten, die jeweils mittels Schmelzverbinden von jeweils überlappenden Abschnitten gebildet werden, wo sich gegenüberliegende Seitenkantenabschnitte eines bauchseitigen Abschnitts und gegenüberliegende Seitenkantenabschnitte eines Rückseitenabschnitts überlappen, und jeder seitenabdichtende Abschnitt Bereiche aufweist, die jeweils ein anderes Gesamtflächengewicht haben als Elemente, die den überlappenden Abschnitt bilden, wobei:
jeder der überlappenden Abschnitte aus mehreren Elementen mit jeweiligen Flächengewichten besteht; und
je größer ein Gesamtflächengewicht der jeweiligen Flächengewichte der mehreren Elemente in einem Bereich des seitenabdichtenden Abschnitts ist, desto kleiner wird ein schmelzverbundener Bereich in diesem Bereich des seitenabdichtenden Abschnitts gemacht.

2. Anziehbarer absorbierender Artikel nach Anspruch 1, wobei:
der anziehbare absorbierende Artikel aufweist: einen wesentlich verlängerten absorbierenden Hauptteil mit einer oberen Schicht, einer hinteren Schicht, und einem absorbierenden Kern zwischen der oberen und der hinteren Schicht; und einer Ummantelung, die sich auf der Seite der hinteren Schicht des absorbierenden Hauptkörpers befindet;
die Ummantelung umfasst eine Außenschichtbahn, die eine Außenfläche des anziehbaren absorbierenden Artikels bildet, und eine Innenschichtbahn, die sich auf einer Innenseite der Außenschichtbahn befindet; und
die überlappenden Abschnitte bestehen aus der Ummantelung.

3. Anziehbarer absorbierender Artikel nach Anspruch 2, wobei die Ummantelung einen durch Falten eines sich nach außen erstreckenden Abschnitts entweder der Außenschichtbahn oder der Innenschichtbahn, der sich nach außen längs dem absorbierenden Körper erstreckt, in Richtung der Innenschichtbahn geformten gefalteten Abschnitt hat.

4. Anziehbarer absorbierender Artikel nach Anspruch 2 oder 3, wobei der überlappende Abschnitt derart ausgestaltet ist, dass er neben den die Ummantelung bildenden Elementen ein weiteres Element aufweist.

5. Anziehbarer absorbierender Artikel nach Anspruch 1, wobei:
die Bereiche in jedem seitenabdichtenden Abschnitt mit jeweils unterschiedlichem Gesamtflächengewicht dadurch gebildet werden, indem eine Anzahl der den überlappenden Abschnitt bildenden Elemente variiert wird; und
je größer die Anzahl der Elemente ist, die den überlappenden Abschnitt ausmachen, desto größer wird das Gesamtflächengewicht der jeweiligen Flächengewichte der mehreren Elemente in jedem Bereich.

6. Anziehbarer absorbierender Artikel nach einem der Ansprüche 1 bis 5, wobei eine Schmelzfestigkeit des seitenabdichtenden Abschnitts bei 5 N/30 mm bis 40 N/30 mm liegt, gemessen mit dem hier beschriebenen Verfahren.

7. Anziehbarer absorbierender Artikel nach einem der Ansprüche 1 bis 6, wobei das Verhältnis (Fmin/Fmax) einer Mindestschmelzfestigkeit des seitenabdichtenden Abschnitts zu einer Maximalschmelzfestigkeit des seitenabdichtenden Abschnitts bei 2/3 < Fmin/Fmax ≤ 1 liegt, gemessen mit dem hier beschriebenen Verfahren.

8. Anziehbarer absorbierender Artikel nach einem der Ansprüche 1 bis 7, wobei die jeweiligen Schmelzkomponenten der Elemente, die den überlappenden Abschnitt ausmachen, Komponenten der gleichen Serie wie Polypropylen, Polyethylen oder Polyester sind.

## Revendications

1. Article absorbant à enfiler, comprenant une paire de sections d'étanchéification latérale formées chacune par le collage par fusion de sections de chevauchement respectives où des sections bord latéral opposées d'une section côté estomac et des sections bord latéral opposées d'une section côté dos se chevauchent les unes sur les autres, chaque section d'étanchéification latérale comprenant des régions ayant chacune un poids unitaire total différent d'éléments qui composent la section de chevauchement, dans lequel :
chacune des sections de chevauchement est composée d'une pluralité d'éléments ayant des poids unitaires respectifs ; et
plus un poids unitaire total des poids unitaires respectifs de la pluralité d'éléments est élevé dans une région de la section d'étanchéification latérale, plus une zone collée par fusion est petite dans cette région de la section d'étanchéification latérale.

2. Article absorbant à enfiler selon la revendication 1, dans lequel :
l'article absorbant à enfiler comprend : un corps absorbant sensiblement allongé comportant une feuille supérieure, une feuille arrière et une partie centrale absorbante située entre la feuille supérieure et la feuille arrière ; et une enveloppe disposée du côté de la feuille arrière du corps absorbant ;
l'enveloppe comprend une feuille de couche externe formant une surface externe de l'article absorbant à enfiler, et une feuille de couche interne disposée sur un côté interne de la feuille de couche externe ; et
les sections de chevauchement sont composées de l'enveloppe.

3. Article absorbant à enfiler selon la revendication 2, dans lequel l'enveloppe comporte une section repliée formée par le repliement, vers la feuille de couche interne, d'une partie s'étendant vers l'extérieur de la feuille de couche externe ou de la feuille de couche interne qui s'étend longitudinalement vers l'extérieur à partir du corps absorbant.

4. Article absorbant à enfiler selon la revendication 2 ou 3, dans lequel la section de chevauchement est conçue pour inclure un élément en plus des éléments formant l'enveloppe.

5. Article absorbant à enfiler selon la revendication 1, dans lequel :
lesdites régions dans chaque section d'étanchéification latérale ayant respectivement différents poids unitaires totaux sont formées en faisant varier un certain nombre desdits éléments qui composent la section de chevauchement ; et
plus le nombre desdits éléments qui composent la section de chevauchement est élevé, plus le poids unitaire total des poids unitaires respectifs de la pluralité des éléments augmente dans chaque région.

6. Article absorbant à enfiler selon l'une quelconque des revendications 1 à 5, dans lequel une résistance de collage par fusion de la section d'étanchéification latérale est de 5 N/30 mm à 40 N/30 mm, et est mesurée selon la méthode décrite dans la description.

7. Article absorbant à enfiler selon l'une quelconque des revendications 1 à 6, dans lequel un rapport (Fmin/Fmax) entre une résistance minimale de collage par fusion de la section d'étanchéification latérale et une résistance maximale de collage par fusion de la section d'étanchéification latérale est 2/3 < Fmin/Fmax ≤ 1, et est mesuré selon la méthode décrite dans la description.

8. Article absorbant à enfiler selon l'une quelconque des revendications 1 à 7, dans lequel les composants respectifs de collage par fusion desdits éléments qui composent la section de chevauchement sont des composants appartenant à une même série de polypropylène, de polyéthylène ou de polyester.
